# FASCICULE DE BREVET EUROPEEN

(11) **EP 1 170 288 B1**
(45) Date de publication et mention de la délivrance du brevet: **11.08.2004**
(21) Numéro de dépôt: 01401712.3
(22) Date de dépôt: 29.06.2001
(51) Int. Cl.: C07D 233/24, C07D 295/12, C07D 319/20, C07D 233/50, C07D 209/08, C07D 405/12, C07D 401/04, C07D 405/04

(54) **Dérivés de diphenylurée et leur utilisation en tant qu'antagonistes alpha2/5-HT2c**
Diphenylharnstoffderivate und deren Verwendung als alpha2/5-HT2c Antagonisten
Diphenylureum derivatives and their use as alpha2/5-HT2c antagonists

(30) Priorité: 29.06.2000 FR 0008378
(43) Date de publication de la demande: 09.01.2002
(73) Titulaire: LES LABORATOIRES SERVIER, 92200 Neuilly sur Seine (FR)
(72) Inventeur: Lavielle, Gilbert, 78170 La Celle Saint Cloud (FR); Muller, Olivier, 95300 Ennery (FR); Millan, Mark, 78230 Le Pecq (FR); Dekeyne, Anne, 78470 Saint Remy Les Chevreuses (FR); Brocco, Mauricette, 75003 Paris (FR)

(56) Documents cités:
- WO-A-95/01976
- WO-A-95/06044
- WO-A-96/11930
- WO-A-98/24785

## Description

La présente invention concerne de nouveaux dérivés de diphénylurée, leur procédé de préparation et les compositions pharmaceutiques qui les contiennent.

L'invention concerne également leur utilisation en tant que ligands mixtes α₂/5-HT_{2c}.

Des composés présentant une structure de diphénylurée ont été décrits dans les demandes JP 11130750 pour leurs caractères antagonistes sérotoninergiques, WO 9506044 et WO 9824785 pour leurs propriétés antagonistes 5-HT, et dans la demande WO 9932436 pour leur utilisation comme inhibiteurs de R.A.F. Kinase.

Le cortex frontal joue un rôle essentiel dans les processus qui contrôlent les fonctions altérées dans les désordres psychiatriques. En particulier, il est maintenant admis que la perturbation de la transmission monoaminergique est fortement impliquée dans l'étiologie de ces différents troubles. Par exemple dans le cas de la dépression, l'activité monoaminergique est diminuée au niveau des régions corticolimbiques.

Parmi les différentes classes d'auto- et hétérorécepteurs de monoamines impliqués dans les mécanismes de régulation, les récepteurs α₂-A.R. (Auto Récepteurs) et 5-HT_{2c} ont montré une importance capitale. Ces deux sous-types réceptoriels agissent dans le même sens en inhibant la transmission dopaminergique et adrénergique. D'une part un rétrocontrôle est exercé par les récepteurs α₂-A.R., sur les neurones noradrénergiques (J. Pharmacol. Exp. Ther., 1994, 270, 958), et d'autre part, les récepteurs 5-HT_{2c} exercent un contrôle inhibiteur sur la transmission dopaminergique et noradrénergique (Neuropharmacology, 1997, 36, 609).

Des composés se liant à l'un ou l'autre de ces sous-types réceptoriels ont montré leur potentiel, par le passé, dans le traitement de plusieurs pathologies.

Ainsi le rôle bénéfique de composés antagonistes α₂ a été étudié dans le traitement des troubles cognitifs (J. Pharmacol., 1992, 6, 376), de la maladie de Parkinson (CNS Drugs, 1998, 10, 189), des troubles de la libido et des dysfonctionnements sexuels (J. Pharmacol., 1997, 11, 72). De la même façon des composés antagonistes des récepteurs 5HT_{2c} ont montré leur utilité dans le traitement des dysfonctionnements sexuels (réf. J. Pharmacol., ibid.) de la maladie de Parkinson (Drug News Perspect., 1999, 12, 477), mais aussi de l'anxiété (Br. J. Pharmacol., 1996, 117, 427) et de la schizophrénie (Neurosci. Lett., 1996, 181, 65).

Des composés possédant un caractère double d'antagonistes α₂-A.R. et 5-HT_{2c} peuvent être d'une grande utilité pour les cliniciens, afin d'obtenir, avec l'administration d'un même composé, une action considérablement renforcée, par un effet de synergie, au niveau de la restauration de la neurotransmission. Ce type de composé présente de plus un avantage considérable par rapport à l'administration de deux produits différents.

Les composés de l'invention présentent une structure originale qui leur confère ce caractère d'antagonistes doubles α₂/5-HT_{2c} et sont donc utiles dans le traitement de la dépression, de l'anxiété, de la schizophrénie, de la maladie de Parkinson, des troubles cognitifs, des troubles de la libido et des dysfonctionnements sexuels, des troubles du sommeil, de l'abus de drogue, et des troubles du comportement impulsif.

La présente invention concerne les composés de formule (I): 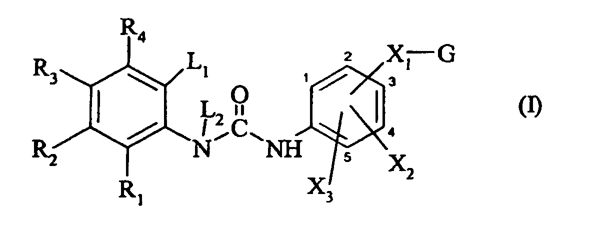 dans laquelle :
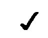 R₁, R₂, R₃, R₄ représentent indépendamment un atome d'hydrogène, d'halogène, un groupement alkyle, alkoxy, hydroxy, alkylthio, mercapto, cyano, amino (éventuellement substitué par un ou deux groupements alkyle), nitro, carboxy, alkoxycarbonyle, aminocarbonyle (éventuellement substitué par un ou deux groupements alkyle) ou carbamoyle,
   ou bien R₃ et R₄, forment ensemble avec les atomes de carbone auxquels ils sont reliés, un cycle phényle et L₁, L₂ forment ensemble un groupement -CH₂-CH₂-,
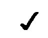 L₁ et L₂ représentent chacun un atome d'hydrogène ou forment ensemble un groupement -CH₂-CH₂-,
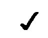 X₁, relié à la position 2 ou 3 du cycle aromatique, représente une liaison et dans ce cas X₂ représente un atome d'hydrogène, d'halogène, ou un groupement alkyle, alkoxy, hydroxy, nitro, cyano ou amino (éventuellement substitué par un ou deux groupements alkyle),
   ou bien,
   X₁ et X₂ forment ensemble un groupement cycloalkyle (C₄-C₇) avec deux carbones adjacents auxquels ils sont reliés en position 2, 3 ou 4 du cycle aromatique, dans lequel un ou deux groupements -CH₂- du cycle cycloalkyle sont éventuellement remplacés par un atome d'oxygène ou un groupement NH, ou N-Alk' (avec Alk' représentant un groupement alkyle) et dans lequel un atome de carbone du groupement cycloalkyle est substitué par le groupe G,
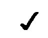 X₃ représente un atome d'hydrogène, d'halogène, ou un groupement alkyle, alkoxy, hydroxy, nitro, cyano, ou amino (éventuellement substitué par un ou deux groupements alkyle),
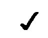 G représente un groupement choisi parmi : 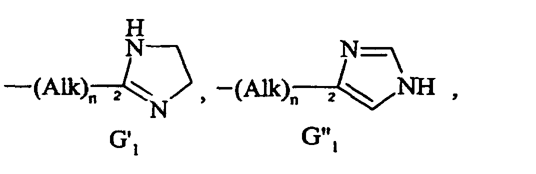 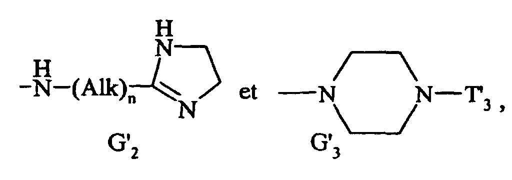 dans lequel T'₃ est un groupement hétéroaryle éventuellement substitué, ou hétéroarylalkyle éventuellement substitué, Alk représente un groupement alkylène (C₁-C₆) linéaire ou ramifié, et n vaut 0 ou 1,
étant entendu que :
- le terme alkyle désigne un groupement linéaire ou ramifié, contenant de 1 à 6 atomes de carbone,
- le terme alkoxy désigne un groupement alkyle-oxy, linéaire ou ramifié contenant de 1 à 6 atomes de carbone,
- le terme aryle désigne un groupement phényle, naphtyle, ou biphényle,
- le terme hétéroaryle désigne un groupement monocyclique aromatique, ou bicyclique dans lequel au moins un des cycles est aromatique, contenant de 5 à 11 chaînons, et 1 à 5 hétéroatomes choisis parmi azote, oxygène et soufre,
- l'expression "éventuellement substitué" associée aux groupements aryle, arylalkyle, hétéroaryle, et hétéroarylalkyle, signifie que ces groupements sont non substitués ou substitués sur la partie cyclique par un ou plusieurs atomes d'halogène et/ou groupements alkyle, alkoxy, hydroxy, mercapto, alkylthio, cyano, amino (éventuellement substitué par un ou deux groupements alkyle), nitro, carboxy, alkoxycarbonyle, aminocarbonyle (éventuellement substitué par un ou deux groupements alkyle), ou carbamoyle, étant entendu que les groupements hétéroaryle et hétéroarylalkyle peuvent en plus être substitués par un groupement oxo,
leurs énantiomères, diastéréoisomères, ainsi que leurs sels d'addition à un acide ou à une base pharmaceutiquement acceptable.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer l'hydroxyde de sodium, l'hydroxyde de potassium, la triéthylamine, la tertbutylamine, etc...

Dans les composés préférés de formule (I), R₁ et R₄ représentent chacun un atome d'hydrogène.

Dans les composés de formule (I), R₂ et R₃ sont avantageusement choisis parmi un atome d'halogène et un groupement alkyle.

Un aspect avantageux de l'invention concerne les composés de formule (I) pour lesquels X₁ est relié à la position 2 du cycle phényle.

Des composés préférés de l'invention sont ceux pour lesquels X₁ représente une liaison et X₂ représente un atome d'halogène, ou un groupement alkyle ou alkoxy.

Un autre aspect avantageux de l'invention concerne les composés de formule (I) pour lesquels X₃ représente un atome d'hydrogène.

D'autres composés préférés de l'invention sont ceux pour lesquels X₁ et X₂ forment ensemble avec les deux carbones en position 2 et 3 du cycle aromatique auxquels ils sont reliés, un groupement cycloalkyle (C₄-C₇), par exemple un groupement cyclopentyle.

Le groupement aryle préféré de l'invention est le groupement phényle.

Parmi les composés préférés de l'invention on peut citer plus particulièrement les :
◆ *N*-(3-Chloro-4-méthylphényl)-*N*'-{3-[4-(2,3-dihydro-1,4-benzodioxin-2-ylméthyl)-1-pipérazinyl]phényl} urée,
◆ *N*-[4-Chloro-3-(4,5-dihydro-1*H*-imidazol-2-ylamino)phényl]-*N*'-(3-chloro-4-méthylphényl)urée,
◆ *N*-(3-Chloro-4-méthylphényl)-*N*'-[2-(1*H*-imidazol-4-yl)-indan-5-yl]urée,
◆ *N*-{3-[4-(2,3-Dihydro-1,4-benzodioxin-2-ylméthyl)-1-pipérazinyl]phényl}-*N*'-(3,4-diméthylphényl)urée,

L'invention s'étend également au procédé de préparation des composés de formule (I).

Un procédé de préparation des composés de formule (I) est caractérisé en ce que l'on utilise comme produit de départ une amine aromatique de formule (II) : 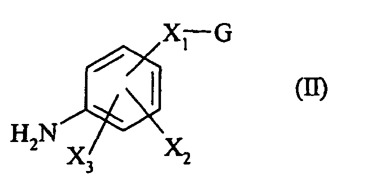 dans laquelle X₁, X₂, X₃ et G sont tels que définis dans la formule (I),
que l'on condense par chauffage en milieu basique sur un dérivé de formule (III) : 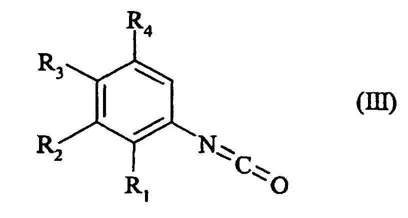 dans laquelle R₁, R₂, R₃ et R₄ sont tels que définis dans la formule (I),
pour conduire au composé de formule (I/a): 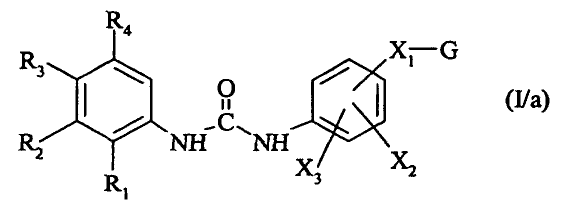 cas particulier des composés de formule (I) pour lequel R₁, R₂, R₃, R₄, X₁, X₂, X₃ et G sont tels que définis précédemment,
étant entendu que l'isocyanate de formule (III) est soit commercial, soit préparé selon des modes opératoires connus, par exemple à partir de l'acide carboxylique correspondant, par réaction avec l'azidure de sodium et réarrangement de l'acyle azide obtenu,
composés de formule (I/a),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

Un autre procédé de préparation des composés de formule (I) est caractérisé en ce que l'on utilise comme produit de départ une amine de formule (IV): 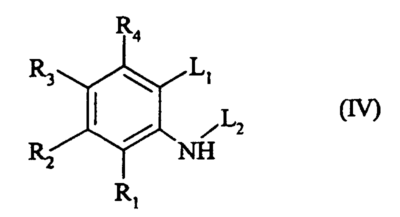 dans laquelle L₁, L₂, R₁, R₂, R₃ et R₄ sont tels que définis dans la formule (I),
que l'on condense par chauffage en milieu basique sur un composé de formule (V) : 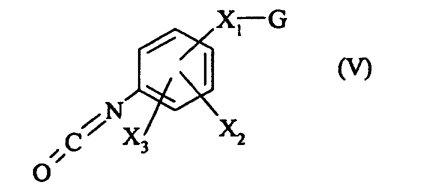 dans laquelle X₁, X₂, X₃ et G sont tels que définis dans la formule (I),
pour conduire au composé de formule (I/b): 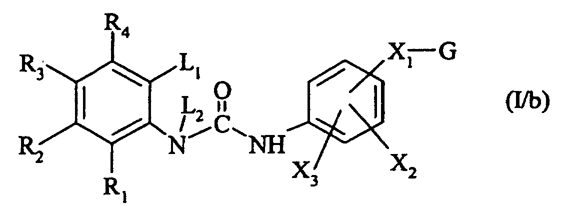 cas particulier des composés de formule (I) pour lequel R₁, R₂, R₃, R₄, L₁, L₂, X₁, X₂, X₃ et G sont tels que définis précédemment,
étant entendu que l'isocyanate de formule (V) est soit commercial, soit préparé selon des modes opératoires connus, notamment à partir de l'acide carboxylique correspondant, par réaction avec l'azidure de sodium et réarrangement de l'azidure d'acyle obtenu,
composés de formule (I/b),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

Un autre procédé de préparation des composés de formule (I) est caractérisé en ce que l'on utilise comme produit de départ une amine de formule (VI) : 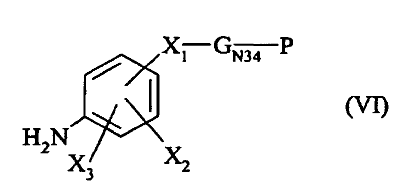 dans laquelle X₁, X₂, et X₃ sont tels que définis dans la formule (I), G_{N34} représente un groupement NH, ou un groupement 1-pipérazinyle ou 4-pipéridinyle, et P représente un atome d'hydrogène ou un groupement protecteur de la fonction amine,
que l'on condense par chauffage en milieu basique sur un composé de formule (III) : 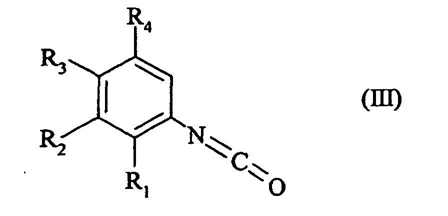 dans laquelle R₁, R₂, R₃ et R₄ sont tels que définis dans la formule (I),
pour conduire au composé de formule (VII) : 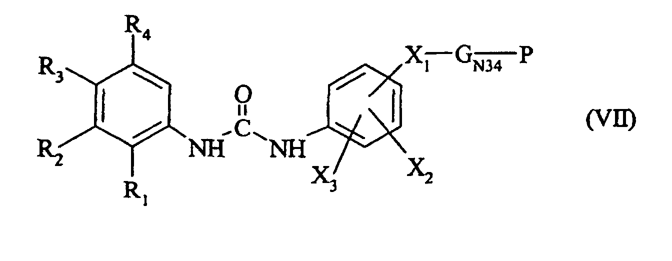 dans laquelle R₁, R₂, R₃, R₄, X₁, X₂, X₃, G_{N34} et P sont tels que définis précédemment,
composés de formule (VII),
- qui, lorsque G_{N34} représente un groupement 1-pipérazinyle ou 4-pipéridinyle, après déprotection éventuelle de la fonction amine, est soumis à une réaction de substitution en milieu basique, de façon à conduire au composé de formule (I/c) : 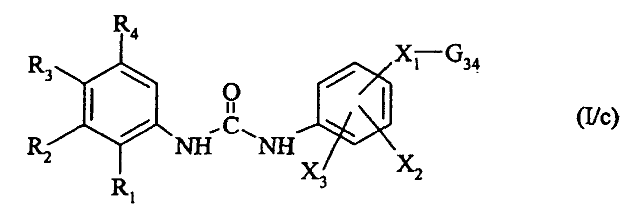 cas particulier des composés de formule (I) pour lequel R₁, R₂, R₃, R₄, X₁, X₂ et X₃ sont tels que définis précédemment et G₃₄ représente un groupement G₃ ou G₄ tel que défini dans la formule (I),
ou bien
- qui, lorsque G_{N34} représente un groupement NH, après déprotection éventuelle, est condensé sur le thiophosgène pour conduire au composé de formule (VIII) : 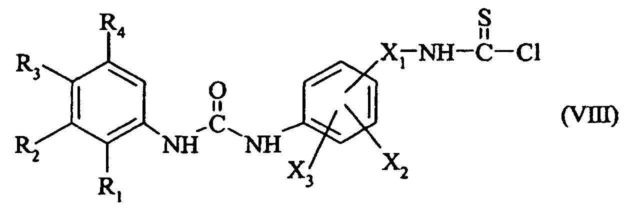 dans laquelle R₁, R₂, R₃, R₄, X₁, X₂ et X₃ sont tels que définis précédemment,
qui est soumis à l'action de l'éthylènediamine pour conduire au composé de formule (IX) : 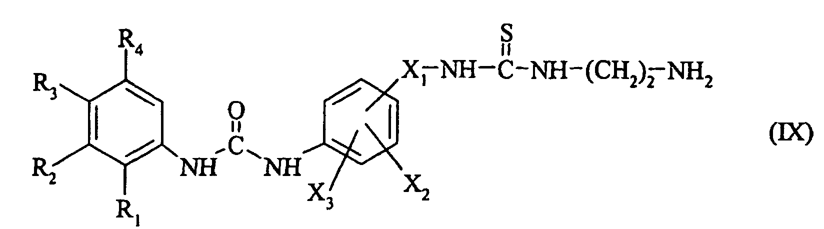 dans laquelle R₁, R₂, R₃, R₄, X₁, X₂ et X₃ sont tels que définis précédemment,
composé de formule (IX) qui subit une réaction de cyclisation intramoléculaire catalysée par un dérivé du palladium, pour conduire au composé de formule (I/d): 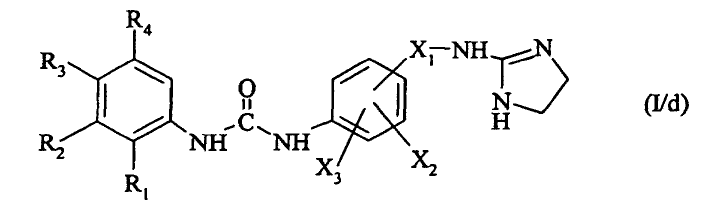 cas particulier des composés de formule (I) pour lequel R₁, R₂, R₃, R₄, X₁, X₂ et X₃ sont tels que définis précédemment,
composés de formule (I/c) et (I/d),
- qui peuvent être, le cas échéant, purifiés selon une technique classique de purification,
- dont on sépare, le cas échéant, les isomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

Les produits de départ utilisés sont des produits connus ou préparés selon des modes opératoires connus.

### Préparation A : 3-(4,5-Dihydro-1H-imidazol-2-ylméthyl)aniline

### Stade 1 : 2-(3-Nitrobenzyl)-4,5-dihydro-1H-imidazole, chlorhydrate

Un mélange de 30,7 mmol (5 g) de 3-nitrophénylacétonitrile et de 30 mmol (7,2 g) de paratoluènesulfonate d'éthylènediamine est chauffé à 100°C pendant 1 heure. Après refroidissement à 20°C, le milieu est hydrolysé par 100 ml d'une solution aqueuse de soude 5M puis extrait au dichlorométhane. Les phases organiques sont séchées sur sulfate de magnésium et concentrées. Le résidu obtenu est transformé en chlorhydrate par action d'une solution d'éthanol chlorhydrique pour conduire au produit attendu.

### Stade 2 : 3-(4,5-Dihydro-1H-imidazol-2-ylméthyl)aniline

Une solution de 22,7 mmol (5,5 g) du produit décrit au stade précédent dans un mélange de 100 ml d'éthanol et 10 ml d'eau, est agitée sous atmosphère d'hydrogène, en présence de 0,5 g de palladium sur charbon à 10 %. Lorsque l'absorption d'hydrogène est terminée, le milieu réactionnel est filtré et concentré pour conduire au produit attendu.

### Préparation B : 3-[1-(4,5-Dihydro-1H-imidazol-2-yl)éthyl]aniline

### Stade 1 : 2-(3-Nitrophényl)propanenitrile

Un mélange de 62 mmol (10 g) de 3-nitrophénylacétonitrile, de 1,11 mol (100 g) de diméthylcarbonate et 3,1 mmol (0,43 g) de carbonate de potassium est chauffé à 170°C pendant 6 heures dans un autoclave. Après refroidissement, 200 ml de dichlorométhane sont ajoutés, et la phase organique est lavée par 100 ml d'eau puis par 100 ml d'une solution aqueuse saturée en chlorure de sodium. La phase organique est séchée sur sulfate de magnésium et concentrée. Le résidu obtenu est purifié par chromatographie sur gel de silice en utilisant comme éluant un mélange cyclohexane/acétate d'éthyle 90/10 pour conduire au produit attendu.

### Stade 2 : 2-[1-(3-Nitrophényl)éthyl]-4,5-dihydro-1H-imidazole

Le produit attendu est obtenu selon le procédé décrit dans la préparation A, stade 1, en utilisant comme produit de départ le composé décrit au stade précédent.

### Stade 3 : 3-[1-(4,5-Dihydro-1H-imidazol-2-yl)éthyl]aniline

Le produit attendu est obtenu selon le procédé décrit dans la préparation A, stade 2, en utilisant comme produit de départ le composé décrit au stade précédent.

### Préparation C : 3-[1-(4,5-Dihydro-1H-imidazol-2-yl)-1-méthyléthyl]aniline

### Stade 1 : 2-Méthyl-2-(3-nitrophényl)propanenitrile

A une solution, agitée vigoureusement, de 123 mmol (20 g) de 3-nitrophénylacétonitrile et de 369 mmol (46,5 g) de sulfate de diméthyle dans 200 ml de diméthylsulfoxyde, sont ajoutés 40 ml de soude à 50 %. Après une heure d'agitation, le milieu réactionnel est dilué par 2 l d'eau et extrait deux fois par 1 l d'éther éthylique. Les phases organiques sont séchées sur sulfate de sodium et concentrées pour conduire au produit attendu.

### Stade 2 : 2-[1-Méthyl-1-(3-nitrophényl)éthyl]-4,5-dihydro-1H-imidazole

Le produit attendu est obtenu selon le procédé décrit dans la préparation A, stade 1, en utilisant comme produit de départ le composé décrit au stade précédent.

### Stade 3 : 3-[1-(4,5-Dihydro-1H-imidazol-2-yl)-1-méthyléthyl]aniline

Le produit attendu est obtenu selon le procédé décrit dans la préparation A, stade 2, en utilisant comme produit de départ le composé décrit au stade précédent.

### Préparation D : 4-Méthyl-3-(4-méthyl-1-pipérazinyl)aniline

### Stade 1 : 4-(2-Méthylphényl)-1-pipérazinecarbaldéhyde

Sous agitation vigoureuse, 437 mmol (77 g) de 2-méthylphénylpipérazine sont ajoutées à une solution de 415 mmol (61,3 g) de trichloroacétaldéhyde dans 400 ml de dibutyléther. Le milieu réactionnel est porté à 80°C pendant 1 heure, et concentré après refroidissement pour conduire au produit attendu.

### Stade 2 : 1-Méthyl-4-(2-méthylphényl)pipérazine

Une solution de 437 mmol (90 g) de dérivé décrit au stade précédent dans 400 ml de tétrahydrofurane est ajoutée à une suspension de 568 mmol (21,6 g) de tétrahydroaluminate de lithium dans 300 ml de tétrahydrofurane. La réaction est agitée 12 heures à 50°C. Après refroidissement, le milieu réactionnel est hydrolysé par 52,5 ml d'eau, puis 48 ml d'une solution aqueuse d'hydroxyde de sodium à 10 % et enfin par 88,5 ml d'eau. Le précipité formé est filtré sur célite, et le filtrat concentré. Le résidu obtenu est repris dans 200 ml d'eau et extrait 3 fois par 250 ml de dichlorométhane. La phase organique est séchée sur sulfate de magnésium et concentrée pour conduire au produit attendu.

### Stade 3 : 1-Méthyl-4-(2-méthyl-5-nitrophényl)pipérazine, chlorhydrate

A une solution de 347 mmol (100 g) d'hydrogénosulfate du composé décrit au stade précédent dans 500 ml d'acide sulfurique concentré, sont ajoutées 416 mmol (64 g) de nitrate de potassium en poudre. L'agitation est maintenue pendant 5 heures, et le milieu réactionnel est versé sur 1200 g de glace, puis neutralisé avec du carbonate de potassium solide et extrait 3 fois par 500 ml d'acétate d'éthyle. Les phases organiques sont séchées et concentrées pour conduire au produit attendu. Le chlorhydrate correspondant est obtenu par action d'une solution d'éthanol chlorhydrique.

### Stade 4 : 4-Méthyl-3-(4-méthyl-1-pipérazinyl)aniline

Le produit attendu est obtenu selon le procédé décrit dans la préparation A, stade 2, en utilisant comme produit de départ le composé décrit au stade précédent.

### Préparation E : 3-[4-(2,3-Dihydro-1,4-benzodioxin-2-ylméthyl)-1-pipérazinyl] aniline

### Stade 1 : 1-(2,3-Dihydro-1,4-benzodioxin-2-ylméthyl)-4-(3-nitrophényl) pipérazine

Une solution de 54,2 mmol (10 g) de 2-chlorométhyl-2,3-dihydro-1,4-benzodioxine, de 54,2 mmol (9,2 g) de 3-nitrophénylpipérazine et de 6 g d'hydrogénocarbonate de potassium dans 100 ml de méthyl-4-pentanone est chauffée à reflux pendant 72 heures. Après refroidissement, le milieu réactionnel est concentré. Le résidu est repris dans 200 ml d'eau et extrait par 200 ml de dichlorométhane. La phase organique est séchée sur sulfate de magnésium, concentrée et purifiée par chromatographie sur gel de silice en utilisant comme éluant un mélange dichlorométhane/méthanol/ammoniaque 99/1/0,1, pour conduire au produit attendu.

### Stade 2 : 3-[4-(2,3-Dihydro-1,4-benzodioxin-2-ylméthyl)-1-pipérazinyl]aniline

Le produit attendu est obtenu selon le procédé décrit dans la préparation A, stade 2, en utilisant comme produit de départ le composé décrit au stade précédent.

### Préparation F : N³-(4,5-Dihydro-1H-imidazol-2-yl)-4-méthyl-1,3-benzènediamine

### Stade 1 : Chlorure de l'acide 2-méthyl-5-nitrophénylcarbamothioique

2,25 l d'eau sont ajoutés à une solution de 130 mmol (20 g) de 2-méthyl-5-nitroaniline dans 375 ml d'acide chlorhydrique concentré. A une température de 0°C, 162 mmol (19 g) de thiophosgène sont coulées en une seule fois. La réaction est agitée vigoureusement pendant 24 heures à température ambiante. Le précipité formé est filtré, puis repris dans l'éther éthylique. La phase organique est lavée à l'eau, séchée sur sulfate de magnésium et concentrée pour conduire au produit attendu.

### Stade 2 : N-(2-Aminoéthyl)-N'-(2-méthyl-5-nitrophényl)thiourée

Une solution de 123 mmol (24 g) du composé décrit au stade précédent dans 1000 ml de toluène est chauffée à 60°C. On ajoute rapidement 246 mmol (8,27 ml) d'éthylènediamine, et le milieu est chauffé à 100°C pendant 3 heures. Après refroidissement, la phase organique est lavée par une solution d'acide chlorhydrique 1M. La phase aqueuse est alcalinisée par la soude concentrée puis extraite au dichlorométhane. La phase organique est lavée à l'eau, séchée sur sulfate de magnésium, concentrée et purifiée par chromatographie sur gel de silice en utilisant comme éluant un mélange dichlorométhane/méthanol/ammoniaque 90/10/1, pour conduire au produit attendu.

### Stade 3 : N-(2-Méthyl-5-nitrophényl)-4,5-dihydro-1H-imidazol-2-amine

A une solution de 63 mmol (16,0 g) du composé décrit au stade précédent, est ajoutée, à 50°C, une solution chaude de 38,8 g d'hydroxyde de potassium dans 135 ml d'eau. Sous agitation vigoureuse, à 80°C, une solution chaude de 72,5 mmol (27,2 g) d'acétate de plomb dans 135 ml d'eau est ajoutée. Après 30 minutes, le milieu réactionnel est filtré sur célite et concentré. Le résidu est repris dans 100 ml d'eau, et le pH est ramené à 10. Après extraction au dichlorométhane, la phase organique est séchée sur sulfate de magnésium et concentrée pour conduire au composé attendu.

### Stade 4 : N³-(4,5-Dihydro-1H-imidazol-2-yl)-4-méthyl-1,3-benzènediamine

Le produit attendu est obtenu selon le procédé décrit dans la préparation A, stade 2, en utilisant comme produit de départ le composé décrit au stade précédent.

### Préparation G : N¹-(4,5-Dihydro-1H-imidazol-2-yl)-1,3-benzènediamine

### Stade 1 : N-(3-Nitrophényl)-4,5-dihydro-1H-imidazol-2-amine

Le produit attendu est obtenu selon le procédé décrit dans la préparation F, stade 2, en utilisant comme produit de départ le 3-nitrophénylisothiocyanate.

### Stade 2 : N¹-(4,5-Dihydro-1H-imidazol-2-yl)-1,3-benzènediamine

Le produit attendu est obtenu selon le procédé décrit dans la préparation A, stade 2, en utilisant comme produit de départ le composé décrit au stade précédent.

### Préparation H : 2-Méthoxy-5-(4-méthyl-1-pipérazinyl)benzoyl azide

Une solution de 25 mmol (5,3 g) de dichlorophosphate de phényle dans 100 ml de dichlorométhane est ajoutée à une solution de 20 mmol (5 g) d'acide 4-méthoxy-3-(4-méthyl-1-pipérazinyl)benzoïque (décrit dans J. Med. Chem., 1994, 37, p.2255) et de 50 mmol (3,25 g) d'azidure de sodium dans 4,05 ml de pyridine. Après 12 heures d'agitation à température ambiante, la phase organique est lavée par 100 ml d'eau, séchée sur sulfate de magnésium et concentrée pour conduire au produit attendu.

### EXEMPLE 1 : N-(3-Chloro-4-méthylphényl)-N'-[3-(4,5-dihydro-1H-imidazol-2-ylméthyl)phényl]urée, chlorhydrate

Une solution de 4,7 mmol (1 g) du composé décrit dans la préparation A et de 4,7 mmol (0,79 g) de 3-chloro-4-méthylphénylisocyanate dans 50 ml de diméthylformamide est chauffée pendant 2 heures à 100°C. Après refroidissement, le milieu réactionnel est concentré. Le résidu obtenu est repris dans 200 ml de dichlorométhane, et le précipité obtenu est filtré et purifié par chromatographie sur gel de silice en utilisant comme éluant un mélange dichlorométhane/méthanol/ammoniaque 90/10/1, pour conduire au produit attendu. Le chlorhydrate correspondant est obtenu par action d'une solution d'éthanol chlorhydrique.
Point de fusion : 227-229°C

| Microanalyse élémentaire : C₁₈H₁₉ClN₄O, HCl | | | | |
|---|---|---|---|---|
| | C | H | N | Cl |
| % Calculé | 57,00 | 5,31 | 14,77 | 9,35 |
| % Trouvé | 56,56 | 5,41 | 14,32 | 9,59 |

### EXEMPLE 2 : N-(3-Chloro-4-méthylphényl)-N'-{3-[1-(4,5-dihydro-1H-imidazol-2-yl)éthyl]phényl}urée, chlorhydrate

Le produit attendu est obtenu selon le procédé décrit dans l'exemple 1, en remplaçant le produit décrit dans la préparation A par le composé décrit dans la préparation B.
Point de fusion : 100-102°C

| | C | H | N | Cl |
|---|---|---|---|---|
| % Calculé | 58,02 | 5,64 | 14,24 | 9,01 |
| % Trouvé | 58,07 | 5,95 | 13,55 | 8,91 |

### EXEMPLE 3: N-(3-Chloro-4-méthylphényl)-N'-{3-[1-(4,5-dihydro-1H-imidazol-2-yl)-1-méthyléthyl]phényl}urée, chlorhydrate

Le produit attendu est obtenu selon le procédé décrit dans l'exemple 1 en remplaçant, le produit décrit dans la préparation A par le composé décrit dans la préparation C.
Point de fusion : 232-233°C

| | C | H | N | Cl |
|---|---|---|---|---|
| % Calculé | 58,97 | 5,94 | 13,75 | 8,70 |
| % Trouvé | 58,32 | 6,11 | 13,13 | 9,11 |

### EXEMPLE 6: N-(3-Chloro-4-méthylphényl)-N'-{3-[4-(2,3-dihydro-1,4-benzodioxin-2-ylméthyl)-1-pipérazinyl]phényl}urée, chlorhydrate

Le produit attendu est obtenu en utilisant le procédé décrit dans l'exemple 4 en remplaçant le 4-méthoxy-3-(4-méthyl-1-pipérazinyl)phénylamine par le composé décrit dans la préparation E.
Point de fusion : 180-185°C

| Microanalyse élémentaire : C₂₇H₂₉ClN₄O₃, HCl | | | | |
|---|---|---|---|---|
| | C | H | N | Cl |
| % Calculé | 57,30 | 5,53 | 9,90 | 18,79 |
| % Trouvé | 57,36 | 5,55 | 9,63 | 18,85 |

### EXEMPLE 7: N-(3-Chloro-4-méthylphényl)-N'-[3-(4,5-dihydro-1H-imidazol-2-ylamino)-4-méthylphényl]urée, chlorhydrate

Le produit attendu est obtenu selon le procédé décrit dans l'exemple 1, en remplaçant le produit décrit dans la préparation A par le composé décrit dans la préparation F.
Point de fusion : 252-254°C

| Microanalyse élémentaire : C₁₈H₂₀ClN₅O, HCl | | | | |
|---|---|---|---|---|
| | C | H | N | Cl |
| % Calculé | 54,83 | 5,37 | 17,76 | 8,99 |
| % Trouvé | 54,91 | 5,25 | 17,78 | 9,12 |

### EXEMPLE 8 : N-(3-Chloro-4-méthylphényl)-N'-[3-(4,5-dihydro-1H-imidazol-2-ylamino)phényl]urée, chlorhydrate

Le produit attendu est obtenu selon le procédé décrit dans l'exemple 1, en remplaçant le produit décrit dans la préparation A par le composé décrit dans la préparation G.
Point de fusion : 180-185°C

| Microanalyse élémentaire : C₁₇H₁₈ClN₅O, HCl | | | | |
|---|---|---|---|---|
| | C | H | N | Cl |
| % Calculé | 53,01 | 5,10 | 18,14 | 10,38 |
| % Trouvé | 53,18 | 5,02 | 18,25 | 10,16 |

### EXEMPLE 9 : N-[4-Chloro-3-(4,5-dihydro-1H-imidazol-2-ylamino)phényl]-N'-(3-chloro-4-méthylphényl)urée, chlorhydrate

### Stade a : N-(3-Chloro-4-méthylphényl)-N'-(4-chloro-3-nitrophényl)urée

Une solution de 29,8 mmol (5 g) de 3-chloro-4-méthylphénylisocyanate dans 90 ml de toluène est chauffée à 70°C, et 29,8 mmol (5,15 g) de 4-chloro-3-nitroaniline sont coulées. La solution est portée à reflux pendant 24 heures. Le milieu réactionnel est refroidi à l'aide de glace, et le précipité formé est filtré puis rincé à l'éther éthylique pour conduire au produit attendu.

### Stade b : N-(3-Amino-4-chlorophényl)-N'-(3-chloro-4-méthylphényl)urée

Une solution de 22,2 mmol (7,5 g) du composé décrit au stade précédent dans 80 ml d'un mélange méthanol/tétrahydrofurane est chauffée à 45°C en présence de nickel de Raney. En contrôlant la température, 33,3 mmol (1,61 ml) d'hydrate d'hydrazine sont ajoutées. La température est maintenue à 45°C pendant 30 minutes et 33,3 mmol (1,61 ml) d'hydrate d'hydrazine sont à nouveau ajoutées. Le milieu est agité 30 minutes à reflux. Après refroidissement, le catalyseur est filtré, et le filtrat concentré. Le résidu obtenu est repris dans l'éther éthylique et lavé, pour conduire au produit attendu.

### Stade c : Chlorure de l'acide 2-chloro-5-{[(3-chloro-4-méthylanilino)carbonyl] amino}phénylcarbamothioique

A une suspension de 3,2 mmol (0,32 g) de carbonate de calcium dans un mélange de 15 ml de dichlorométhane et 2,2 ml d'eau, sont ajoutées 3,2 mmol (0,25 ml) de thiophosgène à 5°C. A cette même température, 3,2 mmol (1 g) du composé décrit au stade précédent en solution dans le dichlorométhane sont ajoutées. Après addition de 4,25 mmol (0,36 g) d'hydrogénocarbonate de sodium, la réaction est agitée 15 minutes à température ambiante. Après filtration sur célite, le filtrat est décanté et la phase organique est lavée à l'eau puis par une solution aqueuse saturée de chlorure de sodium. Après séchage sur sulfate de magnésium et filtration, le filtrat est concentré. Le résidu obtenu est repris dans l'éther éthylique et lavé pour conduire au produit attendu.

### Stade d : N-[3-({[(2-Aminoéthyl)amino]carbothioyl}amino)-4-chlorophényl]-N'-(3-chloro-4-méthylphényl)urée

A une solution de 2,2 mmol (0,78 g) du composé décrit à l'étape précédente dans 35 ml de toluène, chauffée à 60°C, sont ajoutées rapidement 4,4 mmol (0,27 ml) d'éthylène diamine. Le milieu réactionnel est porté à 100°C pendant 3 heures. Après refroidissement, la phase organique est lavée par une solution d'acide chlorhydrique 1N (10 ml). La phase aqueuse est alcalinisée par de la soude concentrée puis extraite au dichlorométhane. La phase organique est lavée à l'eau, séchée sur sulfate de magnésium, filtrée et concentrée. Le résidu obtenu est purifié par chromatographie sur gel de silice en utilisant comme éluant un mélange dichlorométhane/méthanol/ammoniaque 90/10/1, pour conduire au produit attendu.

### Stade e : N-[4-Chloro-3-(4,5-dihydro-1H-imidazol-2-ylamino)phényl]-N'-(3-chloro-4-méthylphényl)urée

A une solution de 1,5 mmol (0,63 g) du composé décrit à l'étape précédente dans 10 ml d'éthanol à 50°C, est ajoutée une solution chaude de 16,5 mmol (1,5 g) d'hydroxyde de potassium dans 5,5 ml d'eau. Sous agitation vigoureuse à 80°C, une solution chaude de 1,72 mmol (1,1 g) d'acétate de plomb dans 5,5 ml d'eau est ajoutée au milieu réactionnel. Après 30 minutes, le milieu est filtré sur célite et le filtrat est concentré. Le résidu est repris dans 5 ml d'eau, le pH est amené à 10, et extrait au dichlorométhane. La phase organique est séchée sur sulfate de magnésium, concentrée et purifiée par chromatographie sur gel de silice en utilisant comme éluant un mélange dichlorométhane/méthanol/ammoniaque, pour conduire au produit attendu. Le chlorhydrate correspondant est obtenu par action d'une solution d'éthanol chlorhydrique.
Point de fusion : 255-257°C

| Microanalyse élémentaire : C₁₇H₁₇Cl₂N₅O₃ HCl | | | | |
|---|---|---|---|---|
| | C | H | N | Cl |
| % Calculé | 49,23 | 4,37 | 16,89 | 25,65 |
| % Trouvé | 48,82 | 4,47 | 16,62 | 25,44 |

### EXEMPLE 10 : N-(3-Chloro-4-méthylphényl)-N'-{3-[4-(2,3-dihydro-1,4-benzodioxin-2-ylméthyl)-1-pipérazinyl]-4-méthoxyphényl}urée, chlorhydrate

### Stade a : tert-Butylate de l'acide 4-(5-{[(3-Chloro-4-méthylanilino)carbonyl] amino}-2-méthoxyphényl)-1-pipérazinecarboxylique

Une solution de 34,4 mmol (10 g) de tert-butylate de l'acide 4-(5-amino-2-méthoxyphényl)pipérazine-1-carboxylique (décrit dans J. Med. Chem., 1999, p.202) et de 37,8 mmol (5,9 g) de 3-chloro-4-méthylphénylisocyanate dans 150 ml de toluène est chauffée 2 heures à reflux. Le milieu réactionnel est concentré, et le résidu est repris dans 200 ml d'acide chlorhydrique 4N, puis chauffé à reflux pendant 4 heures. Après refroidissement, le précipité formé est filtré et traité par une solution d'hydroxyde de sodium 2N pour régénérer la base correspondante.

### Stade b : N-(3-Chloro-4-méthylphényl)-N'-{3-[4-(2,3-dihydro-1,4-benzodioxin-2-ylméthyl)-1-pipérazinyl]-4-méthoxyphényl}urée

Une solution de 12,3 mmol (5 g) du produit décrit au stade précédent dans un mélange de 100 ml d'acétonitrile et 100 ml de diéthylcétone est chauffée 48 heures à reflux en présence de 12,3 mmol (2,3 g) de 2-chlorométhyl-2,3-dihydro-1,4-benzodioxine, de 1,3 g d'hydrogénocarbonate de potassium et de 100 mg d'iodure de potassium. Après refroidissement, le milieu est concentré et le résidu obtenu est extrait au dichlorométhane. La phase organique est séchée, concentrée, et purifiée par chromatographie sur gel de silice en utilisant comme éluant un mélange dichlorométhane/méthanol/ammoniaque 97/3/0,3, pour conduire au produit attendu. Le chlorhydrate correspondant est obtenu par action d'une solution d'éthanol chlorhydrique.
Point de fusion : 193-197°C

| Microanalyse élémentaire : C₂₉H₃₁ClN₄O₄, HCl | | | | |
|---|---|---|---|---|
| | C | H | N | Cl |
| % Calculé | 58,23 | 5,88 | 9,58 | 7,29 |
| % Trouvé | 58,95 | 5,72 | 9,82 | 8,08 |

### EXEMPLE 12 : N-{3-[4-(2,3-dihydro-1,4-benzodioxin-2-ylméthyl)-1-pipérazinyl] phényl}-1,2-dihydro-3H-benzo[e]indole-3-carboxamide, dichlorhydrate

A une solution de 3,12 g de 1,2 dihydrobenzo[e]indole dans 300 ml de toluène sont ajoutés 20 ml d'une solution toluènique de phosgène à 20% en poids à 20°C. Après une heure à 50°C, le mélange est porté à 100°C avec bullage d'azote. On ajoute ensuite à 20°C une solution dans le toluène de 6 g du composé décrit dans la préparation E, puis on porte à 80°C 12 heures. Après traitement le résidu est purifié par chromatographie sur gel de silice en utilisant comme éluant un mélange dichlorométhane/méthanol/ammoniaque 99/1/0,1.
Le dichlorhydrate correspondant est obtenu par action d'une solution d'éthanol chlorhydrique.
Point de fusion : 194-196°C

| Microanalyse élémentaire : C₃₂H₃₂N₄O₃,2HCl | | | | |
|---|---|---|---|---|
| | C | H | N | Cl |
| % Calculé | 64,75 | 5,79 | 9,44 | 11,95 |
| % Trouvé | 65,17 | 5,75 | 9,46 | 10,88 |

### EXEMPLE 13 : 6-Chloro-5-fluoro-N-{3-[4-(2,3-dihydro-1,4-benzodioxin-2-ylméthyl)-1-pipérazinyl]-phényl}-1-2,3-dihydroindolecarboxamide, dichlorhydrate

Le produit attendu est obtenu en utilisant le procédé décrit dans l'exemple 12 en remplaçant le 1,2 dihydrobenzo[e]indole par le 6-chloro-5-fluoro-2,3-dihydroindole.
Point de fusion : 202-204°C

| Microanalyse élémentaire : C₂₈H₂₈ClFN₄O₃,2HCl | | | | |
|---|---|---|---|---|
| | C | H | N | Cl |
| % Calculé | 56,43 | 5,07 | 9,40 | 17,85 |
| % Trouvé | 56,22 | 4,92 | 9,40 | 17,98 |

### EXEMPLE 14 : N-{3-[4-(2,3-Dihydro-1,4-benzodioxin-2-ylméthyl)-1-pipérazinyl]-4-méthoxyphényl}-N'-(3,4-diméthylphényl)urée, dichlorhydrate

### Stade 1 : 1-(2,3-Dihydro-benzo[1,4]dioxin-2-ylméthyl)-4-(2-méthoxy-5-nitrophényl)-pipérazine

Le produit attendu est obtenu en utilisant le procédé décrit dans la préparation E en remplaçant la 3-nitrophénylpipérazine par la 2-méthoxy 5-nitrophénylpipérazine.

### Stade 2 : 3-[4-(2,3-Dihydrobenzo[1,4]dioxin-2-ylméthyl)-pipérazin-1-yl]-4-méthoxy-phénylamine

Le produit attendu est obtenu en utilisant le procédé décrit dans la préparation A, stade 2, en utilisant comme produit de départ le composé décrit au stade précédent.

### Stade 3 : N-{3-[4-(2,3-Dihydro-1,4-benzodioxin-2-ylméthyl)-1-pipérazinyl]-4-méthoxyphényl}-N'-(3,4-diméthylphényl)urée, dichlorhydrate

Le produit final est alors obtenu en utilisant le procédé décrit dans l'exemple 4 en remplaçant la 4-méthoxy-3-(4-méthyl-1-pipérazinyl)phénylamine par le composé décrit au stade 2 de cet exemple et en remplaçant le 3-chloro-4-méthylphénylisocyanate par le 3,4-diméthyl phénylisocyanate.
*Point de fusion :* 234-236° C

| *Microanalyse élémentaire* : C₂₉H₃₄N₄O₄,2HCl | | | | |
|---|---|---|---|---|
| | C | H | N | Cl |
| % Calculé | 60,52 | 6,30 | 9,73 | 12,32 |
| % Trouvé | 60,50 | 6,23 | 9,79 | 12,12 |

### EXEMPLE 15 : N-(3-Chloro-4-fluoro-phényl)-N'-{3-[4-(2,3-dihydro-1,4-benzodioxin-2-ylméthyl)-1-pipérazinyl]-4-méthoxy-phényl}urée, chlorhydrate

Le produit attendu est obtenu en utilisant le procédé décrit dans l'exemple 14 en remplaçant le 3,4-diméthyl phénylisocyanate par le 3-chloro-4-fluoro-phénylisocyanate.
*Point de fusion* : 120-130° C

| *Microanalyse élémentaire* : C₂₇H₂₈ClFN₄O₄HCl | | | | |
|---|---|---|---|---|
| | C | H | N | Cl |
| % Calculé | 57,56 | 5,19 | 9,94 | 12,58 |
| % Trouvé | 57,62 | 5,39 | 9,59 | 11,72 |

### EXEMPLE 16 : N-{3-[4-(2,3-Dihydro-1,4-benzodioxin-2-ylméthyl)-1-pipérazinyl] phényl}-N'-(3,4-diméthylphényl)urée, chlorhydrate

Le produit attendu est obtenu en utilisant le procédé décrit dans l'exemple 4 en remplaçant le 4-méthoxy-3-(4-méthyl-1-pipérazinyl)phénylamine par le composé décrit dans la préparation E et en utilisant le 3,4-diméthylphénylisocyanate à la place du 3-chloro-4-méthylphénylisocyanate.
*Point de fusion :* 228-230° C

| *Microanalyse élémentaire* : C₂₈H₃₂N₄O₃,HCl | | | | |
|---|---|---|---|---|
| | C | H | N | Cl |
| % Calculé | 66,07 | 6,53 | 11,01 | 6,96 |
| % Trouvé | 65,80 | 6,50 | 10,96 | 7,37 |

### EXEMPLE 17 : N-(3-Chloro-4-fluoro-phényl)-N'-{3-[4-(2,3-dihydro-1,4-benzodioxin-2-ylméthyl)-1-pipérazinyl]phényl}urée, chlorhydrate

Le produit attendu est obtenu en utilisant le procédé décrit dans l'exemple 4 en remplaçant le 4-méthoxy-3-(4-méthyl-1-pipérazinyl)phénylamine par le composé décrit dans la préparation E et en utilisant le 3-chloro-4-fluorophénylisocyanate à la place du 3-chloro-4-méthylphénylisocyanate.
*Point de fusion :* 132-136°C

| *Microanalyse élémentaire* : C₂₆H₂₆ClFN₄O₃,HCl | | | | |
|---|---|---|---|---|
| | C | H | N | Cl |
| % Calculé | 58,54 | 5,10 | 10,50 | 13,29 |
| % Trouvé | 58,40 | 5,47 | 10,02 | 12,61 |

### EXEMPLE 18 : 6-Chloro-5-méthyl-N-{4[4-(2,3-dihydro-1,4-benzodioxin-2-ylméthyl)-1-pipérazinyl]phényl}-1-2,3-dihydroindolecarboxamide, dichlorhydrate

Le produit attendu est obtenu en utilisant le procédé décrit dans l'exemple 12 en remplaçant le 1,2 dihydro-benzo[e]indole par le 6-chloro-5-méthyl-2,3-dihydroindole.
*Point de fusion :* 174-176° C

| *Microanalyse élémentaire* : C₂₉H₃₁ClN₄O₃,2HCl | | | | |
|---|---|---|---|---|
| | C | H | N | Cl |
| % Calculé | 58,84 | 5,62 | 9,46 | 17,97 |
| % Trouvé | 58,77 | 5,46 | 9,16 | 18,82 |

### EXEMPLE 19 : N-(3-Chloro-4-méthylphényl)-N'-[2-(1H-imidazol-4-yl)-indan-5-yl] urée, chlorhydrate

### Stade 1 : 2-Bromo-1-indan-2-yl-éthanone

On additionne rapidement 10 g de brome pur à 0°C à une solution de 10 g de 2-acétyl indane dans 150 ml de méthanol anhydre. Après une heure à température ambiante, on ajoute 100 ml d'eau et on laisse sous agitation 12 heures. Après extraction, par 2 fois 200 ml d'éther éthylique, lavage de la phase organique par une solution d'hydrogénocarbonate de sodium puis par de l'eau, le produit attendu est séché sur sulfate de magnésium et concentré.

### Stade 2: 4-Indan-2-yl-1H-imidazole

On porte 30 minutes à 160°C un mélange de 5,2 g de 2-bromo-1-indan-2-yl-éthanone et 43,4 ml de formamide. On ajoute ensuite à température ambiante 40 ml d'eau, puis 40 ml d'acide chlorhydrique 1N. La phase aqueuse est lavée à l'aide de dichlorométhane puis neutralisée par de l'ammoniaque. L'extraction par l'acétate d'éthyle conduit après évaporation en produit attendu.

### Stade 3 : 4-(5-Nitro-indan-2-yl)-1H-imidazole

5 g de 4-indan-2-yl-1H-imidazole sont dissous à 0°C dans 140 ml d'acide sulfurique pur puis on additionne par petites portions 1 équivalent de nitrate d'urée en poudre. Le mélange réactionnel est versé sur de la glace, alcalinisé par de la lessive de soude et extrait à l'aide d'acétate d'éthyle. Après évaporation, on obtient le produit attendu.

### Stade 4 : 2-(1H-Imidazol-4-yl)-indan-5-ylamine

Le chlorhydrate du produit obtenu au stade 3 est agité sous atmosphère d'hydrogène en présence de palladium sur charbon à 10 %, dans l'éthanol. Après filtration et concentration du solvant le produit est utilisé tel quel dans l'étape suivante.

### Stade 5 : N-(3-Chloro-4-méthylphényl)-N'-[2-(1H-imidazol-4-yl)-indan-5-yl] urée, chlorhydrate

On chauffe 2 heures à 100°C un mélange de 5,3 g du chlorhydrate du produit obtenu au stade 4, 3,8 g de 3-chloro-4-méthylphénylisocyanate et 150 ml de diméthylformamide. Après évaporation ensuite du solvant et le résidu est purifié par chromatographie sur gel de silice en utilisant comme éluant un mélange dichlorométhane/méthanol/ammoniaque 97/3/0,3.
Le chlorhydrate correspondant est obtenu par action d'une solution d'éthanol chlorhydrique.
*Point de fusion :* 235-237°C

| *Microanalyse élémentaire* : C₂₀H₁₉ClN₄O,1HCl | | | | |
|---|---|---|---|---|
| | C | H | N | Cl |
| % Calculé | 59,56 | 5,00 | 13,89 | 17,58 |
| % Trouvé | 58,95 | 5,17 | 13,36 | 17,01 |

### EXEMPLE 20 : N-[2-(1H-Imidazol-4-yl)indan-5-yl]-N'-(4-méthylsulfanylphényl) urée, chlorhydrate

Le produit attendu est obtenu en utilisant le procédé décrit dans l'exemple 19 en remplaçant le 3-chloro-4-méthylphénylisocyanate par le 4-méthylthiophénylisocyanate.
*Point de fusion :* 243-245° C

| *Microanalyse élémentaire* : C₂₀H₂₀N₄OS,HCl | | | | | |
|---|---|---|---|---|---|
| | C | H | N | Cl | S |
| % Calculé | 59,92 | 5,28 | 13,97 | 8,84 | 8,00 |
| % Trouvé | 59,72 | 5,32 | 13,26 | 8,56 | 7,73 |

### EXEMPLE 21 : N-(3,4-Diméthylphényl)-N'-[2-(1H-imidazol-4-yl)-indan-5-yl]urée, chlorhydrate

Le produit attendu est obtenu en utilisant le procédé décrit dans l'exemple 19 en remplaçant le 3-chloro-4-méthylphénylisocyanate par le 3,4-diméthylphénylisocyanate.
*Point de fusion :* 232-234° C

| *Microanalyse élémentaire* : C₂₁H₂₂N₄O,HCl | | | | |
|---|---|---|---|---|
| | C | H | N | Cl |
| % Calculé | 65,88 | 6,05 | 14,63 | 9,26 |
| % Trouvé | 65,47 | 6,30 | 14,29 | 9,29 |

### EXEMPLE 22 : N-(3-Chloro-4-méthylphényl)-N'-[2-(4,5-dihydro-1H-imidazol-2-yl)-1,2,3,4-tétrahydro-7-isoquinolinyl]urée, chlorhydrate

### Stade 1 : 2-(4,5-Dihydro-1H-imidazol-2-yl)-7-nitro-1,2,3,4-tétrahydro-isoquinoline

On porte 12 heures à reflux un mélange de 10 g de 7-nitro-1,2,3,4-tétrahydro-isoquinoline, 13,7 g d'iodhydrate de 2-méthylsulfanyl-4,5-dihydro-1H-imidazole et 100 ml de méthanol. Par addition d'éther éthylique, un précipité se sépare. Il est repris dans l'eau, neutralisé à l'aide d'hydroxyde de sodium et extrait par le dichlorométhane.

### Stade 2 : 2-(4,5-Dihydro-1H-imidazol-2-yl)-1,2,3,4-tétrahydro-isoquinolin-7-ylamine

On additionne à 40°C 2 ml d'hydrate d'hydrazine à une suspension de 2 g de chlorhydrate du produit obtenu au stade un et 2 g de nickel de Raney dans 50 ml d'éthanol. Après 2 heures à 50°C, le catalyseur est filtré et le solvant évaporé.

### Stade 3 : N-(3-Chloro-4-méthylphényl)-N'-[2-(4,5-dihydro-1H-imidazol-2-yl)-1,2,3,4-tétrahydro-7-isoquinolinyl]urée, chlorhydrate

Le produit attendu est obtenu en utilisant le procédé décrit au stade 5 de l'exemple 19.
*Point de fusion :* 237-239° C

| *Microanalyse élémentaire* : C₂₀H₂₂ClN₅O,HCl | | | | |
|---|---|---|---|---|
| | C | H | N | Cl |
| % Calculé | 57,09 | 5,47 | 16,65 | 16,89 |
| % Trouvé | 57,41 | 5,51 | 16,32 | 16,74 |

### EXEMPLE 23 : N-(3-Chloro-4-méthylphényl)-N'-{3-[2-(1H-imidazol-4-yl)-éthyl]-phényl}urée, chlorhydrate

### Stade 1 : (3-Nitrophényl)-acétaldéhyde

On porte 6 heures à reflux un mélange de 10 g de 2-(3-nitrophényl)éthanol, 25,1 g de 1-hydroxy-1,2-benziodoxol-3(1H)-one 1-oxyde et 300 ml de tétrahydrofurane. Après filtration et concentration du solvant le produit est utilisé tel quel dans l'étape suivante.

### Stade 2 : 2-(3-Nitro-phényl)-1-(1-trityl-1H-imidazol-4-yl)-éthanol

On coule à température ambiante 4,2 ml de bromure d'éthyle magnésium 3M sur 5,48 g de 4-iodo-1-trityl-1H-imidazole en solution dans 30 ml de dichlorométhane. Après 1 heure, 1 g du produit obtenu au stade 1 est coulé en solution dans 20 ml de dichlorométhane. Après hydrolyse par une solution saturée de chlorure d'ammonium, extraction avec du dichlorométhane, puis lavage de la phase organique à l'eau, le solvant est évaporé et le résidu est purifié par chromatographie sur gel de silice en utilisant comme éluant un mélange de dichlorométhane/méthanol 98/2.

### Stade 3 : 4-[2-(3-Nitrophényl)vinyl]-1-trityl-1H-imidazole

On porte 5 heures à reflux un mélange de 12,25 g du produit obtenu au stade 2, 1 g d'acide paratoluénesulfonique et 200 ml de toluéne. Après retour à température ambiante, lavage de la solution toluènique par une solution d'hydroxyde de sodium 0,1 N, puis par de l'eau, séchage sur sulfate de magnésium et concentration, on obtient le produit attendu.

### Stade 4 : 4-[2-(3-Nitrophényl)vinyl]-1H-imidazole

On chauffe 2 heures au reflux un mélange de 1g du produit obtenu au stade 3, 6 ml d'acide chlorhydrique concentré et 150 ml de méthanol. Le solvant est concentré et le résidu repris dans un mélange isopranol/éther éthylique. Le produit attendu est obtenu par filtration du précipité formé.

### Stade 5 : 3-[2-(1H-Imidazol-4-yl)-éthyl]phénylamine

Le chlorhydrate du produit obtenu au stade 4 est agité sous atmosphère d'hydrogène en présence de palladium sur charbon à 10 %, dans l'éthanol à 90 %. Après filtration et concentration du solvant le produit est utilisé tel quel dans l'étape suivante.

### Stade 6 : N-(3-Chloro-4-méthylphényl)-N'-{3-[2-(1H-imidazol-4-yl)-éthyl]-phényl}urée, chlorhydrate

Le produit attendu est obtenu en utilisant le procédé décrit au stade 5 de l'exemple 19.
*Point de fusion :* 237-239° C

| *Microanalyse élémentaire* : C₁₉H₁₉ClN₄O,HCl | | | | |
|---|---|---|---|---|
| | C | H | N | Cl |
| % Calculé | 58,32 | 5,15 | 14,32 | 18,12 |
| % Trouvé | 58,40 | 5,13 | 13,97 | 18,35 |

### EXEMPLE 24 : N-(3-Chloro-4-méthylphényl)-N'-{3-[2-(4,5-dihydro-1H-imidazol-2-yl)-éthyl]phényl}urée, chlorhydrate

### Stade 1 : 3-(3-Nitrophényl)-acrylonitrile

On coule à 0°C 75,5 g de diéthylcyanométhylphosphonate en solution dans le tétrahydrofurane sur une suspension d'hydrure de sodium dans le tétrahydrofurane. Après 30 minutes de contact à température ambiante, on coule 56 g de 3-nitrobenzaldéhyde en solution dans le tétrahydrofurane. Après 1 heure on hydrolyse avec 300 ml d'eau, puis le solvant est concentré. Après extraction avec du dichlorométhane, lavage de la phase organique à l'eau, séchage sur sulfate de magnésium et concentration, le précipité est repris par de l'éther éthylique, et filtré.

### Stade 2 : 3-(3-Nitrophényl)-propionitrile

Dans une bombe, on introduit 3 g du produit obtenu au stade 1, 1,59 g de chlorure de tris-(triphénylphosphine)-rhodium(I) et 90 ml de benzène. L'ensemble est chauffé 5 heures sous 5 bars d'hydrogène à 40°C. Le solvant est évaporé et le résidu est purifié par chromatographie sur gel de silice en utilisant comme éluant le dichlorométhane.

### Stade 3 : 2-[2-(3-Nitrophényl)-éthyl]-4,5-dihydro-1H-imidazole

On chauffe 2 heures à 160°C 1g du produit obtenu au stade 2 avec 1,32 g de paratoluénesulfonate d'éthylénediamine. On ajoute, ensuite, une solution d'hydroxyde de sodium 0,1 N, et on extrait au dichlorométhane. La phase organique est lavée à l'eau, séchée sur sulfate de magnésium et concentrée. Le chlorhydrate correspondant est obtenu par action d'une solution d'éthanol chlorhydrique.

### Stade 4 : 3-[2-(4,5-Dihydro-1H-imidazol-2-yl)-éthyl]-phénylamine

Le chlorhydrate du produit obtenu au stade 3 est agité sous atmosphère d'hydrogène en présence de palladium sur charbon à 10 %, dans l'éthanol à 90 %. Après filtration et concentration du solvant le produit est utilisé tel quel dans l'étape suivante.

### Stade 5 : N-(3-Chloro-4-méthylphényl)-N'-{3-[2-(4,5-dihydro-1H-imidazol-2-yl)-éthyl]phényl}urée, chlorhydrate

Le produit attendu est obtenu en utilisant le procédé décrit au stade 5 de l'exemple 19.
*Point de fusion :* 212-214° C

| *Microanalyse élémentaire* : C₁₉H₂₁ClN₄O,HCl | | | | |
|---|---|---|---|---|
| | C | H | N | Cl |
| % Calculé | 58,02 | 5,64 | 14,24 | 18,03 |
| % Trouvé | 57,88 | 5,69 | 13,98 | 17,93 |

### EXEMPLE 25 : N-(3-Chloro-4-méthylphényl)-N'-[8-(1H-imidazol-4-yl)-5,6,7,8-tétrahydro-2-naphthalenyl]urée, chlorhydrate

### Stade 1 : Trifluorométhanesulfonic acid 7-nitro-3,4,4a,8a-tétrahydro-naphthalen-1-yl ester

Sur une solution de 10 g de 7-nitrotétralone et 11,8 g de 2,6-di-terbutyl-4-méthylpyridine dans 365 ml de dichlorométhane, on coule à 0°C, 9,8 ml d'anhydride trifluorométhanesulfonique. Après 24 heures à température ambiante, concentration à sec puis reprise du résidu dans 200 ml de pentane au reflux 30 minutes, le précipité formé est filtré. La phase organique est lavée par une solution d'acide chlorhydrique 1N puis de l'eau, séchée sur sulfate de magnésium et concentrée.

### Stade 2 : 4-(7-Nitro-3,4,4a,8a-tétrahydro-naphthalen-1-yl)-1-trityl-1H-imidazole

On coule à température ambiante 15,34 ml de bromure d'éthyle magnésium 3M sur 16,74 g de 4-iodo-1-trityl-1H-imidazole en solution dans 250 ml de tétrahydrofurane. Après 1 heure, on coule 76,6 ml d'une solution 1N de chlorure de zinc dans l'éther éthylique. Après 1 heure de contact 12,4 g du produit obtenu au stade 1 en solution dans 100 ml de tétrahydrofurane et 2,22 g de tétrakis(triphénylphosphine)palladium (0) sont ajoutés et l'ensemble est porté au reflux. Après hydrolyse par une solution saturée de chlorure d'ammonium, extraction avec du dichlorométhane, la phase organique est lavée à l'eau. Le solvant est ensuite évaporé et le résidu est purifié par chromatographie sur gel de silice en utilisant comme éluant un mélange de cyclohexane/acétate d'éthyle 80/20.

### Stade 3 : 8-(1-Trityl-1H-imidazol-4-yl)-4a,5,6,7,8,8a-hexahydro-naphthalen-2-ylamine

7,7 g du produit obtenu au stade 2 sont agités sous atmosphère d'hydrogène en présence de palladium sur charbon à 10%, dans un mélange de méthanol/tétrahydrofurane. Après filtration et concentration du solvant le produit est utilisé tel quel dans l'étape suivante

### Stade 4 : 8-(1H-Imidazol-4-yl)-4a,5,6,7,8,8a-hexahydro-naphthalen-2-ylamine

On chauffe 2 heures au reflux un mélange de 7,7 g du produit obtenu au stade 3, 4,2 ml d'acide chlorhydrique concentré et 100 ml de méthanol. Après concentration du solvant, le résidu est repris dans un mélange isopranol/éther éthylique.Le produit attendu est obtenu par filtration du précipité.

### Stade 5 : N-(3-Chloro-4-méthylphényl)-N'-[8-(1H-imidazol-4-yl)-5,6,7,8-tétrahydro-2-naphthalenyl]urée, chlorhydrate

On chauffe 3 heures au reflux un mélange de 0,7 g du produit obtenu au stade 4, 0,55 g de 3-chloro-4-méthylphénylisocyanate et 50 ml de toluène. Le précipité est alors filtré.
Le chlorhydrate correspondant est obtenu par action d'une solution d'éthanol chlorhydrique.
*Point de fusion :* 255-257°C

| *Microanalyse élémentaire* : C₂₁H₂₁ClN₄O,1HCl | | | | |
|---|---|---|---|---|
| | C | H | N | Cl |
| % Calculé | 60,44 | 5,31 | 13,42 | 16,99 |
| % Trouvé | 60,28 | 5,41 | 13,02 | 17,20 |

### EXEMPLE 26 : N-(3-Chloro-4-méthylphényl)-N'-[7-(1H-imidazol-4-yl)-5,6,7,8-tétrahydro-2-naphthalenyl]urée, chlorhydrate

### Stade 1 : 7-Nitro-3,4-dihydro-1H-naphthalen-2-one

On dissout à 0°C 100 g de 2 tétralone dans 460ml d'acide sulfurique pur et on additionne par petites portions 84,6 g de nitrate de potassium en poudre. Le mélange est ensuite versé sur de la glace, et extrait à l'aide de dichlorométhane. Après évaporation du solvant et le résidu est purifié par chromatographie sur gel de silice en utilisant comme éluant un mélange cyclohexane/tétrahydrofurane 90/10.

### Stade 2 : Trifluorométhanesulfonic acid 7-nitro-3,4-dihydro-naphthalen-2-yl ester

Le produit attendu est obtenu en utilisant le procédé décrit au stade 1 de l'exemple 25 en remplaçant la 7 nitrotétralone par 14,3 g du produit obtenu au stade 1.

### Stade 3 : 4-(7-Nitro-3,4-dihydro-naphthalen-2-yl)-1-trityl-1H-imidazole

Le produit attendu est obtenu en utilisant le procédé décrit au stade 2 de l'exemple 25 en remplaçant le trifluorométhanesulfonic acid 7-nitro-3,4,4a,8a-tétrahydro-naphthalen-1-yl ester par 11,2 g du produit obtenu au stade 2.

### Stade 4 : 7-(1-Trityl-1H-imidazol-4-yl)-5,6,7,8-tétrahydro-naphthalen-2-ylamine

5,5 g du produit obtenu au stade 3 sont agités sous atmosphère d'hydrogène en présence de palladium sur charbon à 10 %, dans un mélange de méthanol/tétrahydrofurane. Après filtration et concentration du solvant le produit est utilisé tel quel dans l'étape suivante.

### Stade 5 : 7-(1H-Imidazol-4-yl)-5,6,7,8-tétrahydro-naphthalen-2-ylamine

On chauffe 2 heures au reflux un mélange de 5,5 g du produit obtenu au stade 4, 3,8 ml d'acide chlorhydrique concentré et 100 ml de méthanol. Après concentration du solvant et le résidu est repris dans un mélange acétone/éther éthylique. Le précipité est filtré et conduit au produit attendu.

### Stade 6 : N-(3-Chloro-4-méthylphényl)-N'-[7-(1H-imidazol-4-yl)-5,6,7,8-tétrahydro-2-naphthalenyl]urée, chlorhydrate

On chauffe 3 heures au reflux un mélange de 0,9 g du produit obtenu au stade 4, 0,71 g de 3-chloro-4-méthylphénylisocyanate et 70 ml de toluène. Le précipité est filtré puis recristallisé dans un mélange éthanol/méthanol.
Le chlorhydrate correspondant est obtenu par action d'une solution d'éthanol chlorhydrique.
*Point de fusion :* 206-208°C

| Microanalyse élémentaire : C₂₁H₂₁ClN₄O,1HCl | | | | |
|---|---|---|---|---|
| | C | H | N | Cl |
| % Calculé | 60,44 | 5,31 | 13,42 | 16,99 |
| % Trouvé | 59,84 | 5,17 | 13,06 | 16,88 |

### EXEMPLE 27 : N-(3-Chloro-4-méthylphényl)-N'-[4-(1H-imidazol-4-yl)-chroman-6-yl]urée, chlorhydrate

### Stade 1 : 6-Nitrochroman-4-one

On additionne à -35°C 60 g de 4-chromanone par petite portion sur 385 ml d'acide nitrique à 90 %. On verse sur de la glace, puis on extrait par du dichlorométhane. La phase organique est lavée par une solution saturée d'hydrogénocarbonate de sodium, séchée sur sulfate de magnésium, puis le solvant est évaporé.

### Stade 2 : Trifluorométhanesulfonic acid 6-nitro-2H-chromen-4-yl ester

Le produit attendu est obtenu en utilisant le procédé décrit au stade 1 de l'exemple 25 en remplaçant la 7 nitrotétralone par 20 g du produit obtenu au stade 1.

### Stade 3 : 4-(6-Nitro-2H-chromen-4-yl)-1-trityl-1H-imidazole

Le produit attendu est obtenu en utilisant le procédé décrit au stade 2 de l'exemple 25 en remplaçant le trifluorométhanesulfonic acid 7-nitro-3,4,4a,8a-tétrahydro-naphthalen-1-yl ester par 11,1 g du produit obtenu au stade 2.

### Stade 4 : 4-(1H-Imidazol-4-yl)-chroman-6-ylamine

Le chlorhydrate du produit obtenu au stade 3 est agité sous atmosphère d'hydrogène en présence de palladium sur charbon à 10 %, dans l'éthanol. Après filtration et concentration du solvant le produit est utilisé tel quel dans l'étape suivante.

### Stade 5 : N-(3-Chloro-4-méthylphényl)-N'-(4-(1H-imidazol-4-yl)-chroman-6-yl]urée, chlorhydrate

On chauffe 2 heures au reflux un mélange de 0,76 g du produit obtenu au stade 4, 0,59 g de 3-chloro-4-méthylphénylisocyanate et 70 ml de toluène. Le précipité est filtré puis recristallisé dans un mélange éthanol/méthanol.
Le chlorhydrate correspondant est obtenu par action d'une solution d'éthanol chlorhydrique.
*Point de fusion* : >260°C

| *Microanalyse élémentaire* : C₂₀H₁₉ClN₄O₂,1HCl | | | | |
|---|---|---|---|---|
| | C | H | N | Cl |
| % Calculé | 57,29 | 4,81 | 13,36 | 16,91 |
| % Trouvé | 56,83 | 4,98 | 12,96 | 16,63 |

Les exemples 28 à 30 ont été préparés selon les procédés décrits précédemment.

### EXEMPLE 28 : N-[3-(1H-Imidazol-4-yl)-chroman-6-yl)-1,2-dihydro-3H-benzo[e] indole-3-carboxamide

### EXEMPLE 29 : N-(3-Chloro-4-méthylphényl)-N'-[3-(1H-imidazol-4-yl)-chroman-6-yl]urée

### EXEMPLE 30 : N-(3-Chloro-4-méthylphényl)-N'-[2-(1H-imidazol-4-yl)-1,2,3,4-tétrahydro-7-isoquinolinyl]urée

### ETUDE PHARMACOLOGIQUE

### EXEMPLE A : Test des érections péniennes chez le rat

Le test permet d'évaluer la capacité d'agents pharmacologiques à inhiber les érections péniennes provoquées par l'administration d'un agoniste sélectif 5-HT_{2c}, le RO 60-0175.
Des rats mâles de souche Wistar pesant 120-140 g le jour de l'expérience, sont placés individuellement dans des boîtes d'observation en plexiglass, juste après avoir reçu le composé à tester ou le véhicule. Trente minutes plus tard, les animaux reçoivent le RO 60-0175 (1,25 mg/kg, sous-cutané) et le nombre d'érections effectuées lors des 30 minutes suivantes est comptabilisé.

*Résultats :* Il apparaît que les composés de l'invention sont capables d'inhiber les érections péniennes provoquées par l'administration de l'agoniste sélectif 5-HT_{2c}. Ils possèdent donc un caractère antagoniste au niveau des récepteurs 5-HT_{2c}. A titre d'exemple le composé de l'exemple 6 possède une concentration inhibitrice 50 (IC₅₀) de 0,7 mg/kg.

### EXEMPLE B : Test d'agressivité induite par isolement chez la souris

Les animaux utilisés sont des souris mâles CD-1. Dès leur arrivée, les souris sont isolées en cages individuelles avec nourriture et boisson à volonté. Après une période d'un mois d'isolement, des couples de souris stables en agressivité sont sélectionnés en observant, lorsqu'elles sont mises en présence, la latence, le nombre et la durée des attaques.

Le test a lieu une fois par semaine. Le jour du test chaque souris du couple (résidente et intruse) reçoit une injection sous-cutanée du véhicule (animaux contrôles) ou de produit à tester (animaux traités) à un volume de 10 ml/kg. Après 30 minutes, la souris intruse est introduite dans la cage de la souris résidente. La latence de la première attaque, le nombre et la durée des attaques sont alors mesurées pendant une période de trois minutes.
Un produit est considéré comme spécifiquement antiagressif lorsqu'il diminue le nombre et la durée des attaques à des doses non sédatives.

*Résultats :* Il apparaît que les composés de l'invention diminuent de façon significative le nombre et la durée des attaques. A titre d'exemple, le composé de l'exemple 6 possède une dose inhibitrice 50 (ID₅₀) de 2,5 mg/kg (administration sous cutanée).

### EXEMPLE C : Test d'enfouissement des billes chez la souris

Le test permet d'évaluer la capacité d'agents pharmacologiques à inhiber le comportement spontané d'enfouissement de billes chez la souris, cette inhibition étant prédictive d'actions antidépressive et/ou anti-impulsive.

Des souris mâles de souche NMRI pesant 20 à 25 g le jour de l'expérience, sont placées individuellement dans des boîtes en macrolon contenant 5 cm de sciure et recouvertes par une plaque en plexiglass perforée. Vingt-quatre billes en verre "oeil de chat" sont réparties régulièrement sur la sciure à la périphérie de la boîte. Au terme de 30 minutes d'exploration libre, les animaux sont retirés de la boîte et le nombre de billes enfouies est comptabilisé.

*Résultats :* Il apparaît que les composés de l'invention inhibent le comportement spontané d'enfouissement de billes chez la souris. A titre d'exemple, le composé de l'exemple 6 possède une dose efficace 50 (ED₅₀) de 0,4 mg/kg.

### EXEMPLE D : Détermination de l'affinité pour les récepteurs α₂ adrénergiques chez le rat

L'affinité a été déterminée par des expériences de compétition avec le [³H]-RX 821,002. Les membranes sont préparées à partir de cortex cérébral de rat et incubées en triple avec 0.4 nM de [³H]-RX 821,002 et le composé à tester dans un volume final de 1,0 ml, pendant 60 minutes à 22°C. Le tampon d'incubation contient 50 nM de TRIS-HCl (pH 7,5), 1 mM de EDTA et 100 µM de GppNHp. La fixation non spécifique est déterminée avec 10 µM de phentolamine.

*Analyse de données :* A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est détermine par comptage du liquide de scintillation. Les isothermes de binding sont analysées par régression non-linéaire.

*Résultats :* Les composés de l'invention montrent une activité antagoniste spécifique des récepteurs α₂-adrénergiques avec, par exemple pour le composé de l'Exemple 6 un pKi de 6,7.

### EXEMPLE E : Composition Pharmaceutique

| Formule de préparation pour 1000 comprimés dosés à 10 mg | |
|---|---|
| Composé de l'exemple 6 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 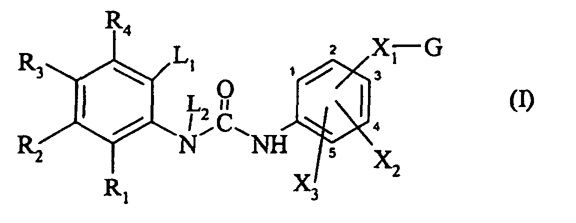 dans laquelle :
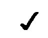 R₁, R₂, R₃, R₄ représentent indépendamment un atome d'hydrogène, d'halogène, un groupement alkyle, alkoxy, hydroxy, alkylthio, mercapto, cyano, amino (éventuellement substitué par un ou deux groupements alkyle), nitro, carboxy, alkoxycarbonyle, aminocarbonyle (éventuellement substitué par un ou deux groupements alkyle) ou carbamoyle,
ou bien R₃ et R₄, forment ensemble avec les atomes de carbone auxquels ils sont reliés, un cycle phényle et L₁, L₂ forment ensemble un groupement -CH₂-CH₂-,
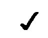 L₁ et L₂ représentent chacun un atome d'hydrogène ou forment ensemble un groupement -CH₂-CH₂-,
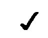 X₁, relié à la position 2 ou 3 du cycle aromatique, représente une liaison et dans ce cas X₂ représente un atome d'hydrogène, d'halogène, ou un groupement alkyle, alkoxy, hydroxy, nitro, cyano ou amino (éventuellement substitué par un ou deux groupements alkyle),
ou bien,
X₁ et X₂ forment ensemble un groupement cycloalkyle (C₄-C₇) avec deux carbones adjacents auxquels ils sont reliés en position 2, 3 ou 4 du cycle aromatique, dans lequel un ou deux groupements -CH₂- du cycle cycloalkyle sont éventuellement remplacés par un atome d'oxygène ou un groupement NH, ou N-Alk' (avec Alk' représentant un groupement alkyle) et dans lequel un atome de carbone du groupement cycloalkyle est substitué par le groupe G,
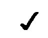 X₃ représente un atome d'hydrogène, d'halogène, ou un groupement alkyle, alkoxy, hydroxy, nitro, cyano, ou amino (éventuellement substitué par un ou deux groupements alkyle),
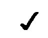 G représente un groupement choisi parmi : 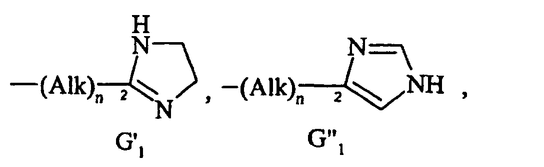 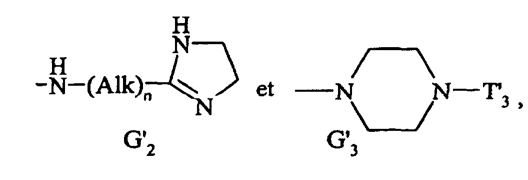 dans lequel T'₃ est un groupement hétéroaryle éventuellement substitué, ou hétéroarylalkyle éventuellement substitué, Alk représente un groupement alkylène (C₁-C₆) linéaire ou ramifié, et n vaut 0 ou 1,
étant entendu que :
- le terme alkyle désigne un groupement linéaire ou ramifié, contenant de 1 à 6 atomes de carbone,
- le terme alkoxy désigne un groupement alkyle-oxy, linéaire ou ramifié contenant de 1 à 6 atomes de carbone,
- le terme aryle désigne un groupement phényle, naphtyle, ou biphényle,
- le terme hétéroaryle désigne un groupement monocyclique aromatique, ou bicyclique dans lequel au moins un des cycles est aromatique, contenant de 5 à 11 chaînons, et 1 à 5 hétéroatomes choisis parmi azote, oxygène et soufre,
- l'expression "éventuellement substitué" associée aux groupements aryle, arylalkyle, hétéroaryle, et hétéroarylalkyle, signifie que ces groupements sont non substitués ou substitués sur la partie cyclique par un ou plusieurs atomes d'halogène et/ou groupements alkyle, alkoxy, hydroxy, mercapto, alkylthio, cyano, amino (éventuellement substitué par un ou deux groupements alkyle), nitro, carboxy, alkoxycarbonyle, aminocarbonyle (éventuellement substitué par un ou deux groupements alkyle), ou carbamoyle, étant entendu que les groupements hétéroaryle et hétéroarylalkyle peuvent en plus être substitués par un groupement oxo,
leurs énantiomères, diastéréoisomères, ainsi que leurs sels d'addition à un acide ou à une base pharmaceutiquement acceptable.

2. Composés de formule (I) selon la revendication 1 pour lesquels L₁ et L₂ représentent chacun un atome d'hydrogène, leurs énantiomères, diastéréoisomères ainsi que leurs sels d'addition à un acide ou à une base pharmaceutiquement acceptable.

3. Composés de formule (I) selon la revendication 1 pour lesquels L₁ et L₂ forment ensemble un groupement -CH₂-CH₂-, leurs énantiomères, diastéréoisomères ainsi que leurs sels d'addition à un acide ou à une base pharmaceutiquement acceptable.

4. Composés de formule (I) selon la revendication 1 pour lesquels R₁ et R₄ représentent chacun un atome d'hydrogène, leurs énantiomères, diastéréoisomères ainsi que leurs sels d'addition à un acide ou à une base pharmaceutiquement acceptable.

5. Composés de formule (I) selon la revendication 1 pour lesquels R₂ et R₃ sont choisis parmi un atome d'halogène et un groupement alkyle, leurs énantiomères, diastéréoisomères ainsi que leurs sels d'addition à un acide ou à une base pharmaceutiquement acceptable.

6. Composés de formule (I) selon la revendication 1 pour lesquels X₁ est relié à la position 2 du cycle phényle, leurs énantiomères, diastéréoisomères ainsi que leurs sels d'addition à un acide ou à une base pharmaceutiquement acceptable.

7. Composés de formule (I) selon la revendication 1 pour lesquels X₁ représente une liaison et X₂ représente un atome d'halogène ou un groupement alkyle ou alkoxy, leurs énantiomères, diastéréoisomères ainsi que leurs sels d'addition à un acide ou à une base pharmaceutiquement acceptable.

8. Composés de formule (I) selon la revendication 1 pour lesquels X₃ représente un atome d'hydrogène, leurs énantiomères, diastéréoisomères ainsi que leurs sels d'addition à un acide ou à une base pharmaceutiquement acceptable.

9. Composés de formule (I) selon la revendication 1 pour lesquels X₁ et X₂ forment ensemble avec les deux carbones en position 2 et 3 du cycle aromatique auxquels ils sont reliés, un groupement cycloalkyle (C₄-C₇), leurs énantiomères, diastéréoisomères ainsi que leurs sels d'addition à un acide ou à une base pharmaceutiquement acceptable.

10. Composé de formule (I) qui est le *N*-(3-chloro-4-méthylphényl)-*N*'-{3-[4-(2,3-dihydro-1,4-benzodioxin-2-ylméthyl)-1-pipérazinyl]phényl}urée, ses énantiomères, diastéréoisomères ainsi que ses sels d'addition à un acide ou à une base pharmaceutiquement acceptable.

11. Composé de formule (I) qui est le *N*-[4-chloro-3-(4,5-dihydro-1*H*-imidazol-2-ylamino) phényl]-*N*'-(3-chloro-4-méthylphényl)urée, ses énantiomères, diastéréoisomères ainsi que ses sels d'addition à un acide ou à une base pharmaceutiquement acceptable.

12. Composé de formule (I) qui est le *N*-(3-chloro-4-méthylphényl)-*N*'-[2-(1H-imidazol-4-yl)-indan-5-yl]urée, ses énantiomères, diastéréoisomères ainsi que ses sels d'addition à un acide ou à une base pharmaceutiquement acceptable.

13. Composé de formule (I) qui est le *N*-{3-[4-(2,3-dihydro-1,4-benzodioxin-2-ylméthyl)-1-pipérazinyl]phényl}-N'-(3,4-diméthylphényl)urée, ses énantiomères, diastéréoisomères ainsi que ses sels d'addition à un acide ou à une base pharmaceutiquement acceptable.

14. Procédé de préparation des composés de formule (I) selon la revendication 1 **caractérisé en ce que** l'on utilise comme produit de départ une amine aromatique de formule (II) : 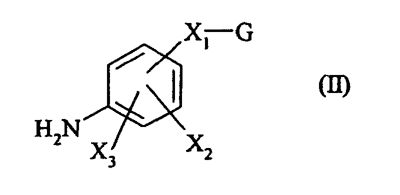 dans laquelle X₁, X₂, X₃ et G sont tels que définis dans la formule (I),
que l'on condense par chauffage en milieu basique sur un dérivé de formule (III) : 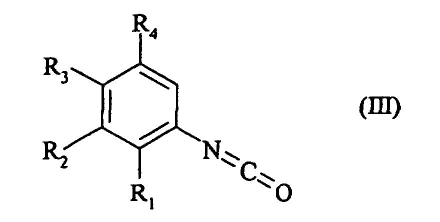 dans laquelle R₁, R₂, R₃ et R₄ sont tels que définis dans la formule (I),
pour conduire au composé de formule (I/a): 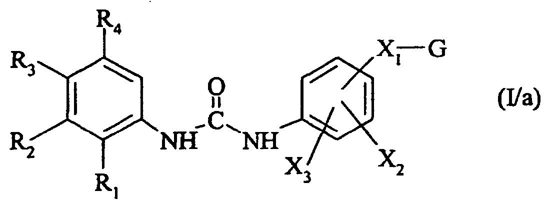 cas particulier des composés de formule (I) pour lequel R₁, R₂, R₃, R₄, X₁, X₂, X₃ et G sont tels que définis précédemment,
étant entendu que l'isocyanate de formule (III) est soit commercial, soit préparé selon des modes opératoires connus,
composés de formule (I/a),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

15. Procédé de préparation des composés de formule (I) selon la revendication 1 **caractérisé en ce que** l'on utilise comme produit de départ une amine de formule (IV) : 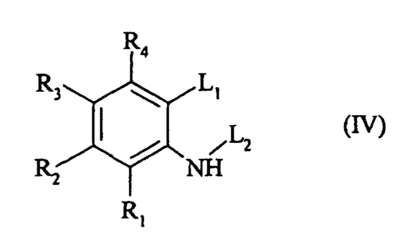 dans laquelle L₁, L₂, R₁, R₂, R₃ et R₄ sont tels que définis dans la formule (I),
que l'on condense par chauffage en milieu basique sur un composé de formule (V) : 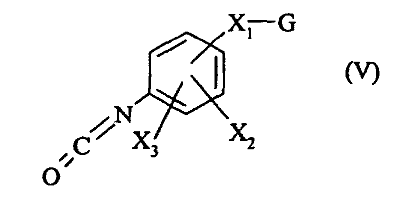 dans laquelle X₁, X₂, X₃ et G sont tels que définis dans la formule (I),
pour conduire au composé de formule (I/b) : 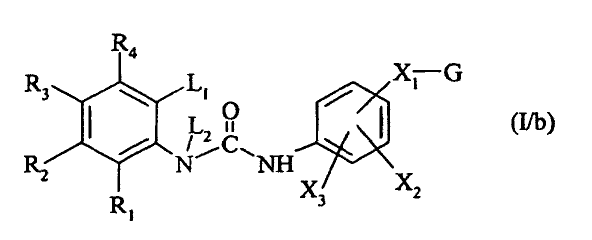 cas particulier des composés de formule (I) pour lequel R₁, R₂, R₃, R₄, L₁, L₂, X₁, X₂, X₃ et G sont tels que définis précédemment,
étant entendu que l'isocyanate de formule (V) est soit commercial, soit préparé selon des modes opératoires connus,
composés de formule (I/b),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

16. Procédé de préparation des composés de formule (I) selon la revendication 1 **caractérisé en ce que** l'on utilise comme produit de départ une amine de formule (VI) : 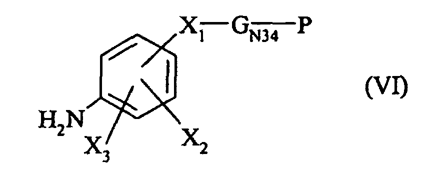 dans laquelle X₁, X₂, et X₃ sont tels que définis dans la formule (I), G_{N34} représente un groupement NH, ou un groupement 1-pipérazinyle ou 4-pipéridinyle, et P représente un atome d'hydrogène ou un groupement protecteur de la fonction amine,
que l'on condense par chauffage en milieu basique sur un composé de formule (III) : 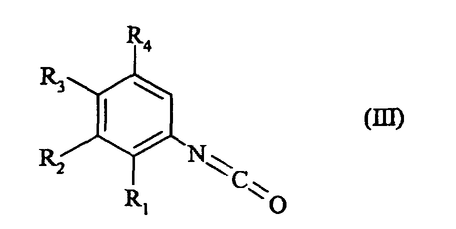 dans laquelle R₁, R₂, R₃ et R₄ sont tels que définis dans la formule (I),
pour conduire au composé de formule (VII) : 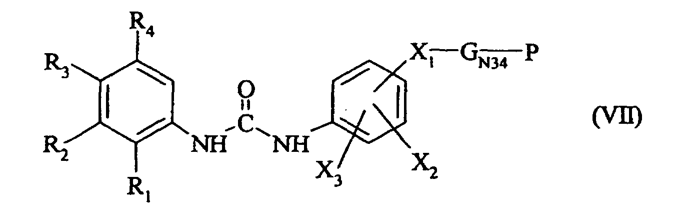 dans laquelle R₁, R₂, R₃, R₄, X₁, X₂, X₃, G_{N34} et P sont tels que définis précédemment,
composés de formule (VII),
- qui, lorsque G_{N34} représente un groupement 1-pipérazinyle ou 4-pipéridinyle, après déprotection éventuelle de la fonction amine, est soumis à une réaction de substitution en milieu basique, de façon à conduire au composé de formule (I/c) : 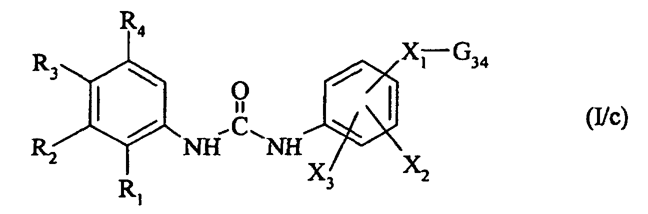 cas particulier des composés de formule (I) pour lequel R₁, R₂, R₃, R₄, X₁, X₂, X₃ sont tels que définis précédemment et G₃₄ représente un groupement G₃ ou G₄ tel que défini dans la formule (I),
ou bien
- qui, lorsque G_{N34} représente un groupement NH, après déprotection éventuelle, est condensé sur le thiophosgène pour conduire au composé de formule (VIII) : 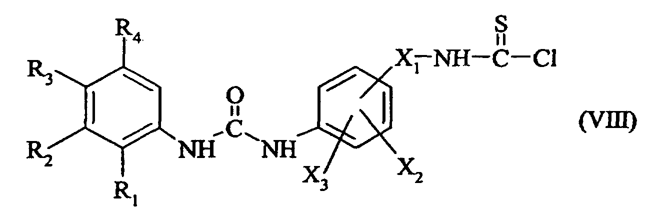 dans laquelle R₁, R₂, R₃, R₄, X₁, X₂ et X₃ sont tels que définis précédemment,
qui est soumis à l'action de l'éthylènediamine pour conduire au composé de formule (IX): 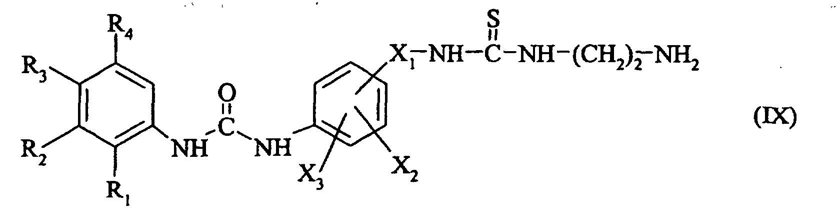 dans laquelle R₁, R₂, R₃, R₄, X₁, X₂ et X₃ sont tels que définis précédemment,
composé de formule (IX) qui subit une réaction de cyclisation intramoléculaire catalysée par un dérivé du palladium, pour conduire au composé de formule (I/d): 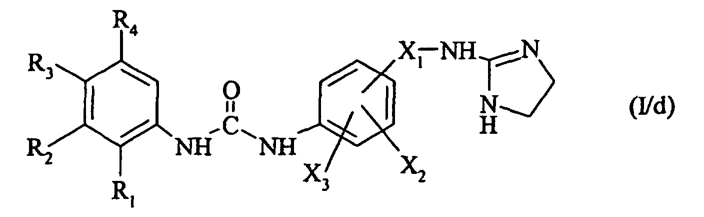 cas particulier des composés de formule (I) pour lequel R₁, R₂, R₃, R₄, X₁, X₂ et X₃ sont tels que définis précédemment,
composés de formule (I/c) et (I/d),
- qui peuvent être, le cas échéant, purifiés selon une technique classique de purification,
- dont on sépare, le cas échéant, les isomères selon une technique classique de séparation,
- que l'on transforme, si on le souhaite, en leurs sels d'addition à un acide ou à une base pharmaceutiquement acceptable.

17. Compositions pharmaceutiques contenant comme principe actif au moins un composé selon l'une quelconque des revendications 1 à 13 seul ou en combinaison avec un ou plusieurs excipients ou véhicules inertes, non toxiques, pharmaceutiquement acceptables.

18. Compositions pharmaceutiques selon la revendication 17 contenant au moins un principe actif selon l'une quelconque des revendications 1 à 13 utiles dans la préparation de médicaments utiles comme antagonistes double α₂/5-HT_{2c}, dans le traitement de la dépression, de l'anxiété, de la schizophrénie, de la maladie de Parkinson, des troubles cognitifs, des troubles de la libido, et des dysfonctionnements sexuels, des troubles du sommeil, de l'abus de drogue, et des troubles du comportement impulsif.

## Patentansprüche

1. Verbindungen der Formel (I): 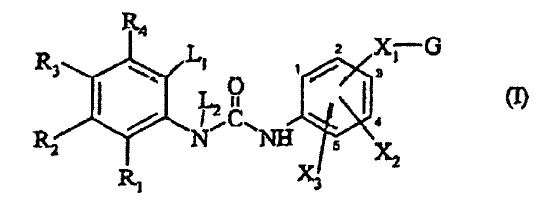 in der:
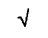 R₁, R₂, R₃, R₄ unabhängig voneinander ein Wasserstoffatom, ein Halogenatom, eine Alkyl-, Alkoxy-, Hydroxy-, Alkylthio-, Mercapto-, Cyano-, (gegebenenfalls durch eine oder zwei Alkylgruppen substituierte) Amino-, Nitro-, Carboxy-, Alkoxycarbonyl-, (gegebenenfalls durch eine oder zwei Alkylgruppen substituierte) Aminocarbonyl- oder Carbamoylgruppe bedeuten,
oder R₃ und R₄ gemeinsam mit den Kohlenstoffatomen, an die sie gebunden sind, einen Phenylring bilden und L₁ und L₂ gemeinsam eine Gruppe -CH₂-CH₂- bilden,
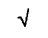 L₁ und L₂ jeweils ein Wasserstoffatom bedeuten oder gemeinsam eine Gruppe -CH₂-CH₂- bilden,
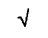 X₁, das in der 2- oder 3-Stellung des aromatischen Rings gebunden ist, eine Bindung bedeutet und in diesem Fall X₂ ein Wasserstoffatom, ein Halogen- atom oder eine Alkyl-, Alkoxy-, Hydroxy-, Nitro-, Cyano- oder (gegebenenfalls durch eine oder zwei Alkylgruppen substituierte) Aminogruppe bedeutet,
oder
X₁ und X₂ gemeinsam mit zwei benachbarten Kohlenstoffatomen, an die sie in der 2-, 3- oder 4-Stellung des aromatischen Rings gebunden sind, eine (C₄-C₇)-Cycloalkylgruppe bilden, wobei eine oder zwei -CH₂-Gruppen des Cycloalkylrings gegebenenfalls durch ein Sauerstoffatom oder eine Gruppe NH oder N-Alk' (worin Alk' eine Alkylgruppe bedeutet) ersetzt sind und bei dem ein Kohlenstoffatom der Cycloalkylgruppe durch die Gruppe G substituiert ist,
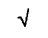 X₃ ein Wasserstoffatom, ein Halogenatom oder eine Alkyl-, Alkoxy-, Hydroxy-, Nitro-, Cyano- oder (gegebenenfalls durch eine oder zwei Alkylgruppen substituierte) Aminogruppe bedeutet,
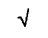 G eine Gruppe bedeutet ausgewählt aus: 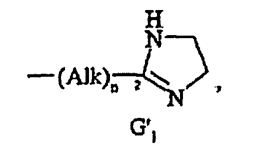 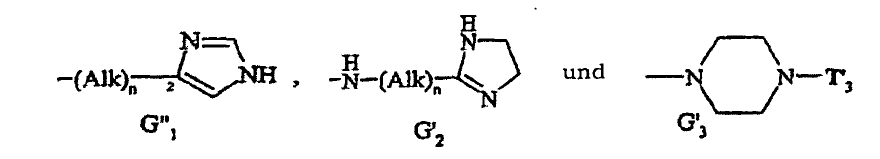 in denen T'₃ eine gegebenenfalls substituierte Heteroarylgruppe oder gegebenenfalls substituierte Heteroarylalkylgruppe, Alk eine geradkettige oder verzweigte (C₁-C₆)-Alkylengruppe und n 0 oder 1 bedeuten,
mit der Maßgabe, daß:
- der Begriff Alkyl für eine geradkettige oder verzweigte Gruppe steht, die 1 bis 6 Kohlenstoffatome enthält,
- der Begriff Alkoxy für eine geradkettige oder verzweigte Alkyloxygruppe steht, die 1 bis 6 Kohlenstoffatome enthält,
- der Begriff Aryl für eine Phenyl-, Naphthyl- oder Biphenylgruppe steht,
- der Begriff Heteroaryl für eine aromatische monocyclische Gruppe oder eine bicyclische Gruppe, bei der mindestens einer der Ringe aromatisch ist, die 5 bis 11 Kettenglieder und 1 bis 5 Heteroatome ausgewählt aus Stickstoff, Sauerstoff und Schwefel enthält, steht,
- der Begriff "gegebenenfalls substituiert" in bezug auf Aryl-, Arylalkyl-, Heteroaryl- und Heteroarylalkylgruppen bedeutet, daß diese Gruppen nicht substituiert sind oder am cyclischen Teil substituiert sind durch ein oder mehrere Halogenatome und/oder Alkyl-, Alkoxy-, Hydroxy-, Mercapto-, Alkylthio-, Cyano-, (gegebenenfalls durch eine oder zwei Alkylgruppen substituierte) Amino-, Nitro-, Carboxy-, Alkoxycarbonyl-, (gegebenenfalls durch eine oder zwei Alkylgruppen substituierte) Aminocarbonyl- oder Carbamoylgruppen, wobei es sich versteht, daß die Heteroaryl- oder Heteroarylalkylgruppen weiterhin durch eine Oxogruppe substituiert sein können,
deren Enantiomere, Diastereoisomere sowie deren Additionssalze mit einer pharmazeutisch annehmbaren Säure oder Base.

2. Verbindungen der Formel (I) nach Anspruch 1, worin L₁ und L₂ jeweils ein Wasserstoffatom bedeuten, deren Enantiomere, Diastereoisomere sowie deren Additionssalze mit einer pharmazeutisch annehmbaren Säure oder Base.

3. Verbindungen der Formel (I) nach Anspruch 1, worin L₁ und L₂ gemeinsam eine Gruppe -CH₂-CH₂- bilden, deren Enantiomere, Diastereoisomere sowie deren Additionssalze mit einer pharmazeutisch annehmbaren Säure oder Base.

4. Verbindungen der Formel (I) nach Anspruch 1, worin R₁ und R₄ jeweils ein Wasserstoffatom bedeuten, deren Enantiomere, Diastereoisomere sowie deren Additionssalze mit einer pharmazeutisch annehmbaren Säure oder Base.

5. Verbindungen der Formel (I) nach Anspruch 1, worin R₂ und R₃ ausgewählt sind aus Halogenatomen und Alkylgruppen, deren Enantiomere, Diastereoisomere sowie deren Additionssalze mit einer pharmazeutisch annehmbaren Säure oder Base.

6. Verbindungen der Formel (I) nach Anspruch 1, worin X₁ in der 2-Stellung des Phenylrings gebunden ist, deren Enantiomere, Diastereoisomere sowie deren Additionssalze mit einer pharmazeutisch annehmbaren Säure oder Base.

7. Verbindungen der Formel (I) nach Anspruch 1, worin X₁ eine Bindung und X₂ ein Halogenatom oder eine Alkyl- oder Alkoxygruppe bedeuten, deren Enantiomere, Diastereoisomere sowie deren Additionssalze mit einer pharmazeutisch annehmbaren Säure oder Base.

8. Verbindungen der Formel (I) nach Anspruch 1, worin X₃ ein Wasserstoff- atom bedeutet, deren Enantiomere, Diastereoisomere sowie deren Additionssalze mit einer pharmazeutisch annehmbaren Säure oder Base.

9. Verbindungen der Formel (I) nach Anspruch 1, worin X₁ und X₂ gemeinsam mit den beiden Kohlenstoffatomen in den 2- und 3-Stellungen des aromatischen Rings, an den sie gebunden sind, eine (C₄-C₇)-Cycloalkylgruppe bilden, deren Enantiomere, Diastereoisomere sowie deren Additionssalze mit einer pharmazeutisch annehmbaren Säure oder Base.

10. Verbindung der Formel (I), nämlich *N*-(3-Chlor-4-methylphenyl)-*N*'-{3-[4-(2,3-dihydro-1,4-benzodioxin-2-ylmethyl)-1-piperazinyl]-phenyl}-harnstoff, dessen Enantiomere, Diastereoisomere sowie deren Additionssalze mit einer pharmazeutisch annehmbaren Säure oder Base.

11. Verbindung der Formel (I), nämlich *N*-[4-Chlor-3-(4,5-dihydro-1*H*-imidazol-2-ylamino)-phenyl]-*N*'-(3-chlor-4-methylphenyl)-harnstoff, dessen Enantiomere, Diastereoisomere sowie deren Additionssalze mit einer pharmazeutisch annehmbaren Säure oder Base.

12. Verbindung der Formel (I), nämlich *N*-(3-Chlor-4-methylphenyl)-*N*'-[2-(1H-imidazol-4-yl)-indan-5-yl]-harnstoff, dessen Enantiomere, Diastereoisomere sowie deren Additionssalze mit einer pharmazeutisch annehmbaren Säure oder Base.

13. Verbindung der Formel (I), nämlich *N*-{3-[4-(2,3-Dihydro-1,4-benzodioxin-2-ylmethyl)-1-piperazinyl]-phenyl}-N'-(3,4-dimethylphenyl)-harnstoff, dessen Enantiomere, Diastereoisomere sowie deren Additionssalze mit einer pharmazeutisch annehmbaren Säure oder Base.

14. Verfahren zur Herstellung der Verbindungen der Formel (I) nach Anspruch 1, **dadurch gekennzeichnet, daß** man als Ausgangsprodukt ein aromatisches Amin der Formel (II) verwendet: 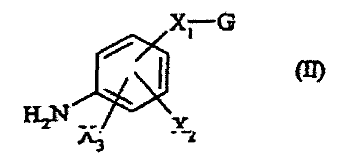 in der X₁, X₂, X₃ und G die bezüglich der Formel (I) angegebenen Bedeutungen besitzen,
welche man durch Erhitzen in basischem Medium mit einem Derivat der Formel (III) kondensiert: 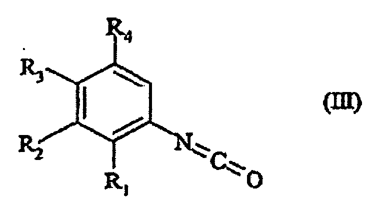 in der R₁, R₂, R₃ und R₄ die bezüglich der Formel (I) angegebenen Bedeutungen besitzen,
zur Bildung der Verbindung der Formel (I/a): 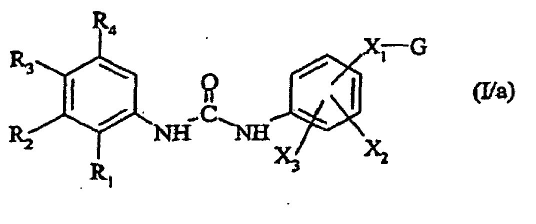 einem Sonderfall der Verbindungen der Formel (I), in der R₁, R₂, R₃, R₄, X₁, X₂, X₃ und G die oben angegebenen Bedeutungen besitzen,
wobei es sich versteht, daß das Isocyanat der Formel (III) entweder im Handel erhältlich ist oder mit Hilfe bekannter Verfahrensweisen hergestellt wird,
welche Verbindungen der Formel (I/a)
- gegebenenfalls mit Hilfe einer klassischen Reinigungsmethode gereinigt werden können,
- man gegebenenfalls mit Hilfe einer klassischen Trennmethode in ihre Isomeren auftrennt,
- gewünschtenfalls in ihre Additionssalze mit einer pharmazeutisch annehmbaren Säure oder Base umwandelt.

15. Verfahren zur Herstellung der Verbindungen der Formel (I) nach Anspruch 1, **dadurch gekennzeichnet, daß** man als Ausgangsprodukt ein Amin der Formel (IV) verwendet: 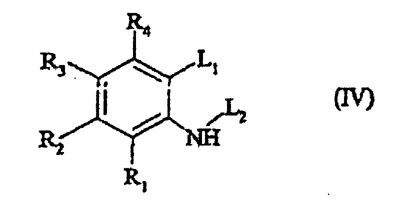 in der L₁, L₂, R₁, R₂, R₃ und R₄ die bezüglich der Formel (I) angegebenen Bedeutungen besitzen,
welches man durch Erhitzen in basischem Medium mit einer Verbindung der Formel (V) kondensiert: 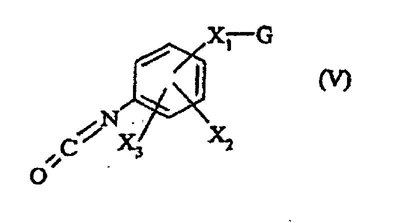 in der X₁, X₂, X₃ und G die bezüglich der Formel (I) angegebenen Bedeutungen besitzen,
zur Bildung der Verbindung der Formel (I/b): 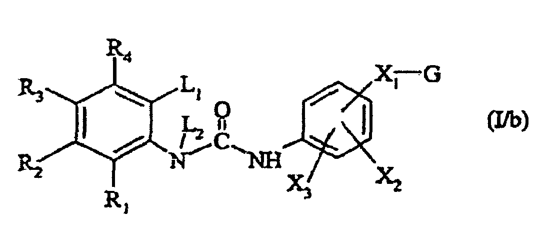 einem Sonderfall der Verbindungen der Formel (I), in der R₁, R₂, R₃, R₄, L₁, L₂, X₁, X₂, X₃ und G die oben angegebenen Bedeutungen besitzen,
wobei es sich versteht, daß das Isocyanat der Formel (V) entweder im Handel erhältlich ist oder mit Hilfe bekannter Verfahrensweisen hergestellt wird,
welche Verbindungen der Formel (I/b) man
- gegebenenfalls mit Hilfe einer klassischen Reinigungsmethode reinigen kann,
- gegebenenfalls mit Hilfe einer klassischen Trennmethode in ihre Isomeren auftrennt
- gewünschtenfalls in ihre Additionssalze mit einer pharmazeutisch annehmbaren Säure oder Base umwandelt.

16. Verfahren zur Herstellung der Verbindungen der Formel (I) nach Anspruch 1, **dadurch gekennzeichnet, daß** man ais Ausgangsprodukt ein Amin der Former (VI) verwendet: 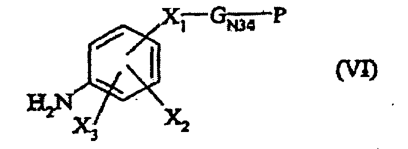 worin X₁, X₂ und X₃ die bezüglich der Formel (I) angegebenen Bedeutungen besitzen, G_{N34} für eine NH-Gruppe oder eine 1-Piperazinyl- oder 4-Piperidinylgruppe steht und P ein Wasserstoffatom oder eine Schutzgruppe für die Aminfunktion bedeutet,
welches man durch Erhitzen in basischem Medium mit einer Verbindung der Formel (III) kondensiert: 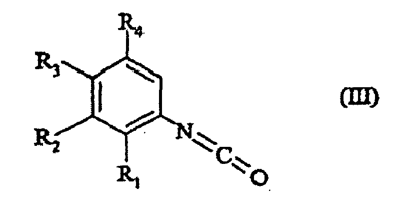 in der R₁, R₂, R₃ und R₄ die bezüglich der Formel (I) angegebenen Bedeutungen besitzen,
zur Bildung der Verbindung der Formel (VII): 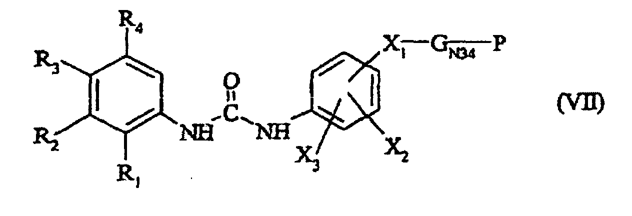 in der R₁, R₂, R₃, R₄, X₁, X₂, X₃, G_{N34} und P die oben angegebenen Bedeutungen besitzen,
welche Verbindungen der Formel (VII)
- man, wenn G_{N34} eine 1-Piperazinyl- oder 4-Piperidinylgruppe bedeutet, nach der eventuellen Abspaltung der Schutzgruppe von der Aminfunktion einer Substitutionsreaktion in basischem Medium unterwirft zur Bildung der Verbindung der Formel (I/c): 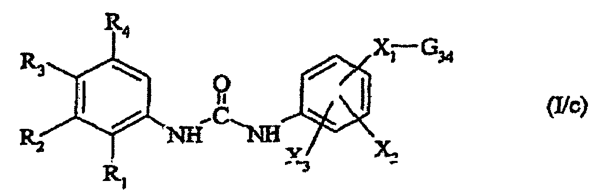 einem Sonderfall der Verbindungen der Formel (I), in der R₁, R₂, R₃, R₄, X₁, X₂ und X₃ die oben angegebenen Bedeutungen besitzen und G₃₄ eine Gruppe G₃ oder G₄ bedeutet, wie sie bezüglich der Formel (I) definiert worden sind,
oder
- wenn G_{N34} eine NH-Gruppe bedeutet, nach der eventuellen Abspaltung der Schutzgruppe mit Thiophosgen kondensiert zur Bildung der Verbindung der Formel (VIII): 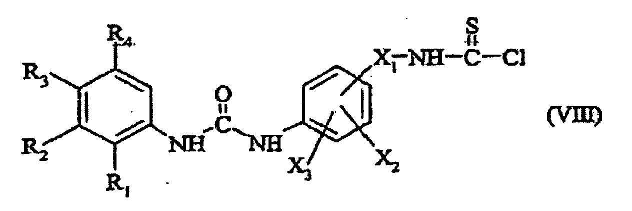 in der R₁, R₂, R₃, R₄, X₁, X₂ und X₃ die oben angegebenen Bedeutungen besitzen.
welche der Einwirkung von Ethylendiamin unterworfen wird zur Bildung der Verbindung der Formel (IX): 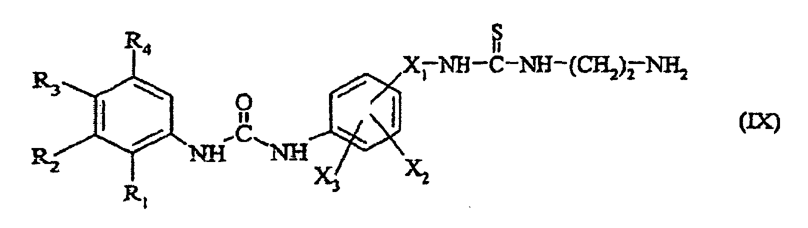 in der R₁, R₂, R₃, R₄, X₁, X₂ und X₃ die oben angegebenen Bedeutungen besitzen,
welche Verbindung der Formel (IX) man einer intramolekularen Cyclisierungsreaktion unterwirft, die mit einem Palladium-Derivat katalysiert wird, zur Bildung der Verbindung der Formel (I/d): 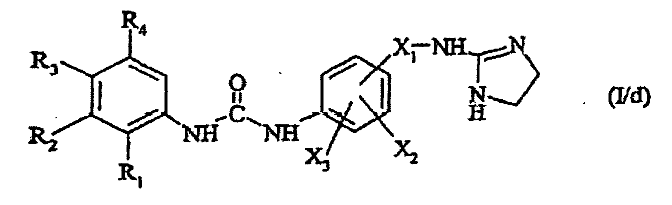 einem Sonderfall der Verbindungen der Formel (I), in der R₁, R₂, R₃, R₄, X₁, X₂ und X₃ die oben angegebenen Bedeutungen besitzen,
welche Verbindungen der Formeln (I/c) und (I/d)
- gegebenenfalls mit Hilfe einer klassischen Reinigungsmethode gereinigt werden können,
- gegebenenfalls mit Hilfe einer klassischen Trennmethode in ihre Isomeren getrennt werden,
- gewünschtenfalls in ihre Additionsalze mit einer pharmazeutisch annehmbaren Säure oder Base überführt werden.

17. Pharmazeutische Zubereitungen enthaltend als Wirkstoff mindestens eine Verbindung nach einem der Ansprüche 1 bis 13 allein oder in Kombination mit einem oder mehreren inerten, nichttoxischen, pharmazeutisch annehmbaren Trägermaterialien oder Bindemitteln.

18. Pharmazeutische Zubereitungen nach Anspruch 17 enthaltend mindestens einen Wirkstoff nach einem der Ansprüche 1 bis 13 zur Herstellung von Arzneimitteln, die nützlich sind als Doppel-α₂/5-HT_{2c}-Antagonisten, bei der Behandlung der Depression, der Angst, der Schizophrenie, der Parkinsonschen Krankheit, von Erkenntnisstörungen, von Störungen der Libido, von sexuellen Dysfunktionen, von Schlafstörungen, dem Drogenmißbrauch und Störungen des impulsiven Verhaltens.

## Claims

1. Compounds of formula (I) : 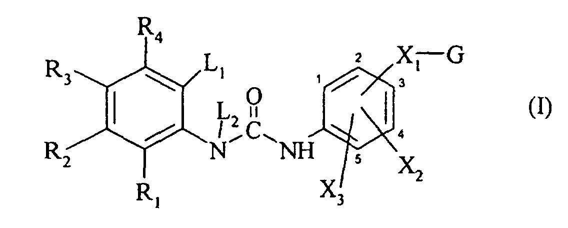 wherein
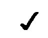 R₁, R₂, R₃ and R₄ independently represent a hydrogen atom, a halogen atom or an alkyl, alkoxy, hydroxy, alkylthio, mercapto, cyano, amino (optionally substituted by one or two alkyl groups), nitro, carboxy, alkoxycarbonyl, aminocarbonyl (optionally substituted by one or two alkyl groups) or carbamoyl group,
or R₃ and R₄, together with the carbon atoms to which they are bonded, form a phenyl ring, and L₁, L₂ together form a -CH₂-CH₂- group,
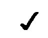 L₁ and L₂ each represents a hydrogen atom or together form a -CH₂-CH₂- group,
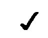 X₁, attached at the 2 or 3 position of the aromatic ring, represents a bond, and in that case X₂ represents a hydrogen atom, a halogen atom, or a group: alkyl, alkoxy, hydroxy, nitro, cyano or amino (optionally substituted by one or two alkyl groups), or,
X₁ and X₂, together with two adjacent carbon atoms to which they are bonded in the 2, 3 or 4 position of the aromatic ring, form a (C₄-C₇)cycloalkyl group wherein one or two -CH₂- groups of the cycloalkyl ring are optionally replaced by an oxygen atom or an NH group or N-Alk' group (wherein Alk' represents an alkyl group) and wherein one carbon atom of the cycloalkyl group is substituted by the group G,
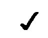 X₃ represents a hydrogen atom, a halogen atom, or a group: alkyl, alkoxy, hydroxy, nitro, cyano, or amino (optionally substituted by one or two alkyl groups),
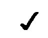 G represents a group selected from : 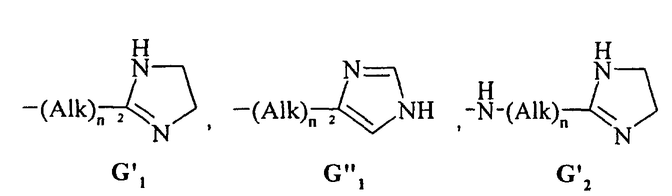 and 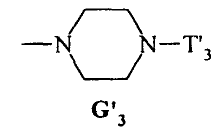 wherein T'₃ is an optionally substituted heteroaryl group or optionally substituted heteroarylalkyl group, Alk represents a linear or branched (C₁-C₆)alkylene group and n is 0 or 1,
wherein :
- the term "alkyl" denotes a linear or branched group containing from 1 to 6 carbon atoms,
- the term "alkoxy" denotes a linear or branched alkyl-oxy group containing from 1 to 6 carbon atoms,
- the term "aryl" denotes a phenyl, naphthyl or biphenyl group,
- the term "heteroaryl" denotes an aromatic monocyclic group, or a bicyclic group in which at least one of the rings is aromatic, containing from 5 to 11 ring members and from 1 to 5 hetero atoms selected from nitrogen, oxygen and sulphur,
- the expression "optionally substituted" associated with the groups aryl, arylalkyl, heteroaryl and heteroarylalkyl denotes that those groups are unsubstituted or substituted on the cyclic moiety by one or more halogen atoms and/or alkyl, alkoxy, hydroxy, mercapto, alkylthio, cyano, amino (optionally substituted by one or two alkyl groups), nitro, carboxy, alkoxycarbonyl, aminocarbonyl (optionally substituted by one or two alkyl groups) or carbamoyl groups, wherein the heteroaryl and heteroarylalkyl groups may in addition be substituted by an oxo group,
enantiomers and diastereoisomers thereof, and also addition salts thereof with a pharmaceutically acceptable acid or base.

2. Compounds of formula (I) according to claim 1, wherein L₁ and L₂ each represents a hydrogen atom, enantiomers and diastereoisomers thereof, and also addition salts thereof with a pharmaceutically acceptable acid or base.

3. Compounds of formula (I) according to claim 1, wherein L₁ and L₂ together form a -CH₂-CH₂- group, enantiomers and diastereoisomers thereof, and also addition salts thereof with a pharmaceutically acceptable acid or base.

4. Compounds of formula (I) according to claim 1, wherein R₁ and R₄ each represents a hydrogen atom, enantiomers and diastereoisomers thereof, and also addition salts thereof with a pharmaceutically acceptable acid or base.

5. Compounds of formula (I) according to claim 1, wherein R₂ and R₃ are selected from a halogen atom and an alkyl group, enantiomers and diastereoisomers thereof, and also addition salts thereof with a pharmaceutically acceptable acid or base.

6. Compounds of formula (I) according to claim 1, wherein X₁ is attached at the 2 position of the phenyl ring, enantiomers and diastereoisomers thereof, and also addition salts thereof with a pharmaceutically acceptable acid or base.

7. Compounds of formula (I) according to claim 1, wherein X₁ represents a bond and X₂ represents a halogen atom or an alkyl or alkoxy group, enantiomers and diastereoisomers thereof, and also addition salts thereof with a pharmaceutically acceptable acid or base.

8. Compounds of formula (I) according to claim 1, wherein X₃ represents a hydrogen atom, enantiomers and diastereoisomers thereof, and also addition salts thereof with a pharmaceutically acceptable acid or base.

9. Compounds of formula (I) according to claim 1, wherein X₁ and X₂, together with the two carbon atoms in the 2 and 3 positions of the aromatic ring to which they are bonded, form a (C₄-C₇)cycloalkyl group, enantiomers and diastereoisomers thereof, and also addition salts thereof with a pharmaceutically acceptable acid or base.

10. Compound of formula (I) that is *N*-(3-chloro-4-methylphenyl)-*N*'-{3-[4-(2,3-dihydro-1,4-benzodioxin-2-ylmethyl)-1-piperazinyl]phenyl}urea, enantiomers and diastereoisomers thereof, and also addition salts thereof with a pharmaceutically acceptable acid or base

11. Compound of formula (I) that is *N*-[4-chloro-3-(4,5-dihydro-1*H*-imidazol-2-ylamino)-phenyl]-*N*'-(3-chloro-4-methylphenyl)urea, enantiomers and diastereoisomers thereof, and also addition salts thereof with a pharmaceutically acceptable acid or base

12. Compound of formula (I) that is *N*-(3-chloro-4-methylphenyl)-*N*'-[2-(1H-imidazol-4-yl)-indan-5-yl]urea, enantiomers and diastereoisomers thereof, and also addition salts thereof with a pharmaceutically acceptable acid or base

13. Compound of formula (I) that is *N*-{3-[4-(2,3-dihydro-1,4-benzodioxin-2-ylmethyl)-1-piperazinyl]phenyl}-*N*'-(3,4-dimethylphenyl)urea, enantiomers and diastereoisomers thereof, and also addition salts thereof with a pharmaceutically acceptable acid or base

14. Process for the preparation of the compounds of formula (I) according to claim 1, **characterised in that** there is used as starting material an aromatic amine of formula (II) : 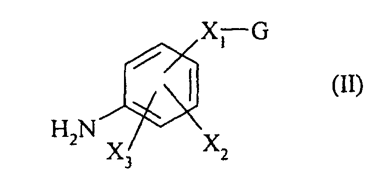 wherein X₁, X₂, X₃ and G are as defined for formula (I),
which is condensed by heating in basic medium with a compound of formula (III) : 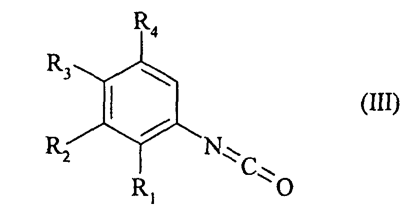 wherein R₁, R₂, R₃ and R₄ are as defined for formula (I),
to yield the compound of formula (I/a) : 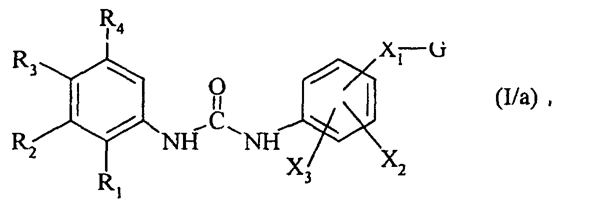 a particular case of the compounds of formula (I) wherein R₁, R₂, R₃, R₄, X₁, X₂, X₃ and G are as defined hereinbefore,
wherein the isocyanate of formula (III) is either commercially available or is prepared according to known procedures,
which compounds of formula (I/a)
- may, if necessary, be purified according to a conventional purification technique,
- are optionally separated into isomers according to a conventional separation technique,
- are, if desired, converted into addition salts with a pharmaceutically acceptable acid or base.

15. Process for the preparation of the compounds of formula (I) according to claim 1, **characterised in that** there is used as starting material an amine of formula (IV) : 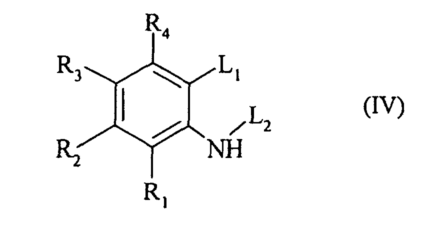 wherein L₁, L₂, R₁, R₂, R₃ and R₄ are as defined for formula (I),
which is condensed by heating in basic medium with a compound of formula (V) : 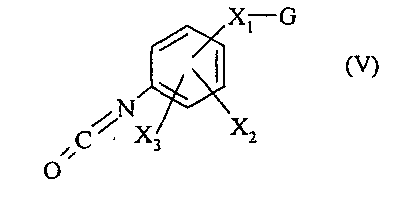 wherein X₁, X₂, X₃ and G are as defined for formula (I),
to yield the compound of formula (I/b) : 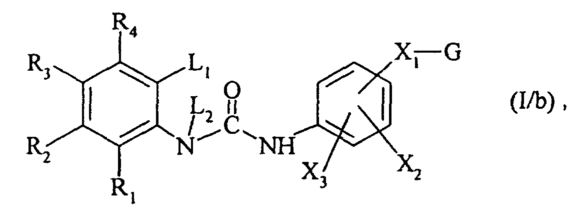 a particular case of the compounds of formula (I) wherein R₁, R₂, R₃, R₄, L₁, L₂, X₁, X₂, X₃ and G are as defined hereinbefore,
wherein the isocyanate of formula (V) is either commercially available or is prepared according to known procedures,
which compounds of formula (I/b)
- may, if necessary, be purified according to a conventional purification technique,
- are optionally separated into isomers according to a conventional separation technique,
- are, if desired, converted into addition salts with a pharmaceutically acceptable acid or base.

16. Process for the preparation of the compounds of formula (I) according to claim 1, **characterised in that** there is used as starting material an amine of formula (VI) : 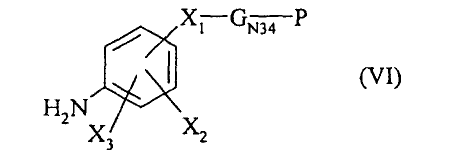 wherein X₁, X₂ and X₃ are as defined for formula (I), G_{N34} represents an NH group or a 1-piperazinyl or 4-piperidinyl group, and P represents a hydrogen atom or a group protecting the amine function,
which is condensed by heating in basic medium with a compound of formula (III) : 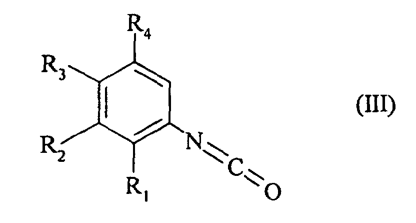 wherein R₁, R₂, R₃ and R₄ are as defined for formula (I),
to yield the compound of formula (VII) : 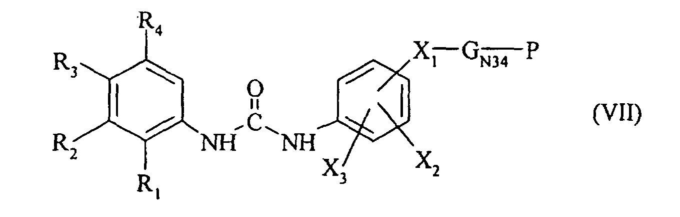 wherein R₁, R₂, R₃, R₄, X₁, X₂, X₃, G_{N34} and P are as defined hereinbefore,
which compound of formula (VII),
- when G_{N34} represents a 1-piperazinyl or 4-piperidinyl group, after deprotection where necessary of the amine function, is subjected to a substitution reaction in basic medium to yield the compound of formula (I/c) : 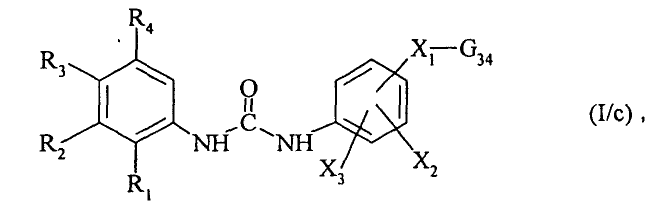 a particular case of the compounds of formula (I) wherein R₁, R₂, R₃, R₄, X₁, X₂ and X₃ are as defined hereinbefore and G₃₄ represents a group G₃ or G₄ as defined for formula (I),
or
- when G_{N34} represents an NH group, after deprotection where necessary, is condensed with thiophosgene to yield the compound of formula (VIII) : 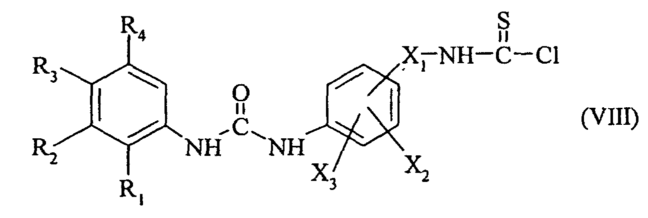
wherein R₁, R₂, R₃, R₄, X₁, X₂ and X₃ are as defined hereinbefore,
which is subjected to the action of ethylenediamine to yield the compound of formula (IX): 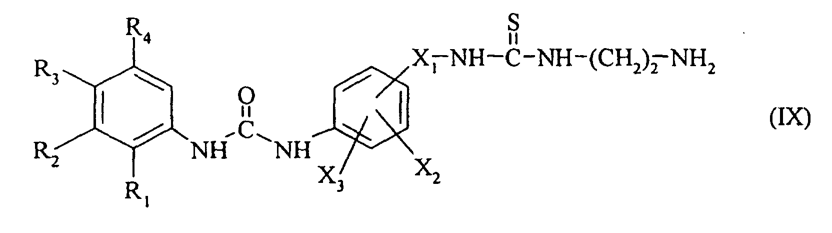 wherein R₁, R₂, R₃, R₄, X₁, X₂ and X₃ are as defined hereinbefore,
which compound of formula (IX) is subjected to an intramolecular cyclisation reaction catalysed by a palladium compound to yield the compound of formula (I/d) : 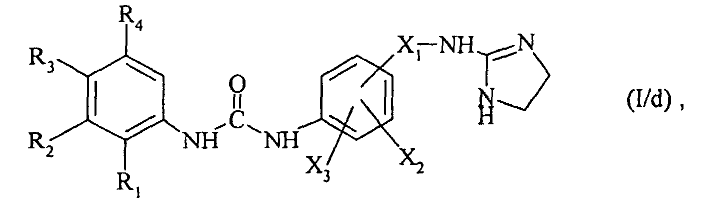 a particular case of the compounds of formula (I) wherein R₁, R₂, R₃, R₄, X₁, X₂ and X₃ are as defined hereinbefore,
which compounds of formulae (I/c) and (I/d),
- may, if necessary, be purified according to a conventional purification technique,
- are optionally separated into isomers according to a conventional separation technique,
- are converted, if desired, into addition salts with a pharmaceutically acceptable acid or base.

17. Pharmaceutical compositions comprising as active ingredient at least one compound according to any one of claims 1 to 13, alone or in combination with one or more pharmaceutically acceptable, inert, non-toxic excipients or carriers.

18. Pharmaceutical compositions according to claim 17, containing at least one active ingredient according to any one of claims 1 to 13, for use in the preparation of medicaments useful as dual α₂/5-HT_{2c} antagonists in the treatment of depression, anxiety, schizophrenia, Parkinson's disease, cognitive disorders, libido disorders, sexual dysfunction, sleep disorders, drug abuse and impulsive behaviour disorders.
